# EUROPÄISCHE PATENTANMELDUNG

(11) **EP 0 703 239 A1**
(43) Veröffentlichungstag der Anmeldung: **27.03.1996**
(21) Anmeldenummer: 95114360.1
(22) Anmeldetag: 13.09.1995
(51) Int. Cl.: C07F 9/40, A61K 31/66, C07F 9/38, C07F 9/48, C07F 9/32, C07F 9/655, C07F 9/6533, C07F 9/59, C07F 9/58, C07F 9/6512

(54) **Aminomethylphosphon- und Aminomethylphosphinsäure-Derivate und deren Verwendung zur Behandlung von degenerativen Gelenkserkrankungen**

(30) Priorität: 19.09.1994 DE 4433244
(71) Anmelder: HOECHST AKTIENGESELLSCHAFT, D-65929 Frankfurt am Main (DE)
(72) Erfinder: Wolf, Erhard, Dr., D-65719 Hofheim (DE); Rossmanith, Erhard, D-65824 Schwalbach (DE); Thorwart, Werner, Dr., D-65239 Hochheim (DE); Raiss, Ruth, Dr., D-60322 Frankfurt (DE); Weithmann, Klaus Ulrich, Dr., D-65719 Hofheim (DE)

(57) **Zusammenfassung**

Verbindungen der Formel I 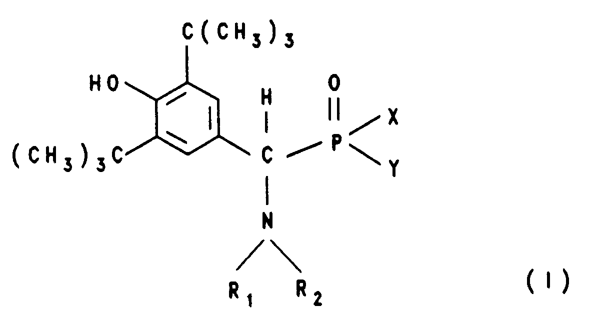
eignen sich zur Herstellung von Arzneimitteln zur Behandlung und Prophylaxe von degenerativen Gelenkserkrankungen, aber auch von Erkrankungen des rheumatischen Formenkreises, bei denen Knorpelabbau zu verzeichnen ist, wie bei der chronischen Polyarthritis, dem Gelenktrauma sowie bei Knorpelschwund nach längerer Immobilisation des Gelenks.

## Beschreibung

Arthrose ist eine degenerative Gelenkserkrankung mit entzündlichen Episoden und progredienter Knorpelzerstörung, die zu Funktionsbeeinträchtigung bis hin zu völliger Versteifung führen kann. Bisher sind zwar die begleitenden Entzündungen und Schmerzzustände bei dieser Erkrankung therapierbar, es stehen aber keine Arzneimittel zur Verfügung, die erwiesenermaßen den fortschreitenden Knorpelabbau aufzuhalten oder zu heilen vermögen. Bekannte Therapeutika der Arthrose sind beispielsweise Mischungen von sulfatierten Glucoseaminoglykanen (Current Therapeutic Research, 40,6 (1986) 1034) oder nichtsteroidale Antiinflammatorika, die jedoch den Knorpelverlust nicht aufzuhalten vermögen.

Wenn auch die Pathogenese der Arthrose noch nicht im Einzelnen aufgeklärt ist, gilt als gesichert, daß die Chondrozyten (Knorpelzellen) maßgeblich an dem verstärkten Matrixverlust beteiligt sind, und daß von den Hauptbestandteilen dieser Matrix vor allem die Proteoglykane (PG) als erste enzymatisch abgebaut werden.

Erfolgsversprechende Ansatzpunkte zur Arthrosetherapie würden demnach solche Pharmaka bieten, die aufgrund ihres Wirkprofils die Proteoglykan-Synthese in Chondrozyten stimulieren und darüberhinaus einem pathologisch beschleunigten Knorpelabbau entgegenwirken. Dabei kann der Abbau der Proteoglykane entweder durch die Hemmung relevanter Matrix-Metalloproteinasen aber auch durch Desaktivierung reaktiver Sauerstoffradikale vermindert werden.

Es wurde nun gefunden, daß die erfindungsgemäßen Aminomethylphosphon- und -phosphinsäure-Derivate die Proteoglykansynthese im Knorpel stimulieren, den Knorpelabbau inhibieren und den durch Sauerstoffradikale ausgelösten Knorpelabbau wirksam vermindern.

Aufgrund ihrer pharmakologischen Eigenschaften eignen sich die erfindungsgemäßen Verbindungen hervorragend zur Behandlung und Prophylaxe von degenerativen Gelenkserkrankungen, aber auch von Erkrankungen des rheumatischen Formenkreises, bei denen Knorpelabbau zu verzeichnen ist, wie bei der chronischen Polyarthritis, dem Gelenktrauma sowie bei Knorpelschwund nach längerer Immobilisation des Gelenks.

Einige der erfindungsgemäß verwendbaren Verbindungen sind bereits beschrieben worden, doch über ihre Verwendung bei degenerativen Gelenkserkrankungen ist nichts bekannt (DE 2 751 943; H. Oswiecimska et al., J. Prakt. Chem. 318 (1976) S. 403 - 408; H. Gross et al., J. Prakt. Chem. 317 (1975) S. 890 - 896; M. G. Pavlichenko et al., Zh Obshch Khim 56 (1986) S. 2000 - 2004; R. K. Ismagilov et al., Zh Obshch Khim 61 (1991) S. 387 - 391).

Die Erfindung betrifft ein Arzneimittel, enthaltend eine Verbindung der Formel I 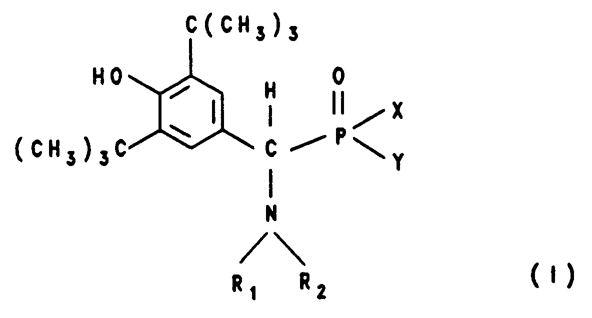
und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₁₂)-Alkyl,
c) (C₁-C₁₂)-Alkyl, ein- bis mehrfach unabhängig voneinander substituiert durch
   1) Phenyl,
   2) Phenyl, ein- bis dreifach unabhängig voneinander substituiert durch
      2.1. Fluor-, Chlor-, Brom- oder Jodatom,
      2.2. (C₁-C₃)-Alkyl,
      2.3. (C₁-C₃)-Alkoxy,
      2.4. Methylendioxy,
      2.5. Nitro,
      2.6. Amino,
      2.7. Amidino,
      2.8. Guanidino,
      2.9. Hydroxy,
      2.10. (C₁-C₆)-Alkoxycarbonyl oder
      2.11. Cyano,
   3) einen monocyclischen 5- oder 6-gliedrigen gesättigten heterocyclischen Ring,
   4) Amino, unsubstituiert oder ein- bis zweimal substituiert durch (C₁-C₃)-Alkyl, oder
   5) -C(O)-O-(C₁-C₃)-Alkyl,
d) Tetralin,
e) Phenyl,
f) Phenyl, ein- bis dreifach unabhängig voneinander substituiert durch
   1) (C₁-C₄)-Alkyl,
   2) (C₁-C₄)-Alkyl, ein- oder mehrfach substituiert durch Fluor-, Chlor-, Brom- oder Jodatom,
   3) Fluor-, Chlor-, Brom- oder Jodatom,
   4) Nitro,
   5) Aminosulfonyl,
   6) Amidino,
   7) Guanidino,
   8) (C₁-C₃)-Alkyoxy,
   9) Methylendioxy,
   10) Hydroxy,
   11) Carboxy,
   12) Cyano,
   13) (C₁-C₃)-Alkyoxycarbonyl,
   14) - CH = CH - COOH,
   15) 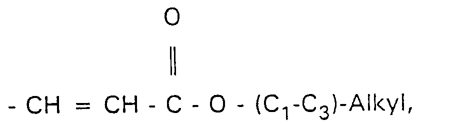
   16) Amino oder
   17) Amino, substituiert durch den Rest der Formel II, 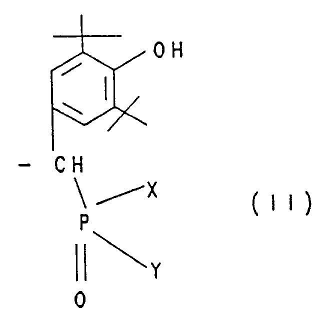 worin X und Y unabhängig voneinander
      17.1. Wasserstoffatom,
      17.2. (C₁-C₆)-Alkyl,
      17.3. (C₁-C₆)-Alkoxy,
      17.4. (C₃-C₆)-Alkenyloxy,
      17.5. Phenoxy oder
      17.6. Hydroxy bedeutet,
g) Naphthyl,
h) Naphthyl, ein- bis mehrfach substituiert wie unter f)1) bis f)17) definiert,
i) Heteroaryl,
k) Heteroaryl, ein- bis mehrfach substituiert durch Phenyl, Benzyl oder wie unter f)1) bis f)17) definiert, steht, oder
l) R¹ und R² bilden zusammen mit dem Stickstoffatom, an das sie gebunden sind, einen 4- bis 7-gliedrigen Ring,
m) R¹ und R² bilden zusammen mit dem Stickstoffatom, an das sie gebunden sind, einen 5- bis 7-gliedrigen Ring, worin ein ringständiges C-Atom durch ein Sauerstoffatom oder ein Stickstoffatom ersetzt ist, oder
n) R¹ und R² bilden zusammen mit dem Stickstoffatom, an das sie gebunden sind, einen 5- bis 7-gliedrigen Ring, worin ein ringständiges C-Atom durch ein Stickstoffatom ersetzt ist und dieses Stickstoffatom ist substituiert durch
   1) Phenyl oder
   2) den Rest der Formel II, worin X und Y unabhängig voneinander die unter f)17.1. bis f)17.6. genannte Bedeutung haben, und
X und Y gleich oder verschieden sind und unabhängig voneinander die unter f)17.1. bis f)17.6. genannte Bedeutung haben;
und einen physiologisch verträglichen Träger.

Bevorzugt ist ein Arzneimittel, enthaltend mindestens eine Verbindung der Formel I und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₁₂)-Alkyl,
c) (C₁-C₁₂)-Alkyl, ein- bis mehrfach unabhängig voneinander substituiert durch
   1) Phenyl,
   2) Phenyl, ein- bis dreifach unabhängig voneinander substituiert durch
      2.1. Fluor-, Chlor-, Brom- oder Jodatom,
      2.2. (C₁-C₃)-Alkyl,
      2.3. (C₁-C₃)-Alkoxy,
      2.4. Methylendioxy,
      2.5. Nitro,
      2.6. Amino,
      2.7. Amidino,
      2.8. Guanidino,
      2.9. Hydroxy,
      2.10. (C₁-C₆)-Alkoxycarbonyl oder
      2.11. Cyano,
   3) Piperazinyl oder
   4) Morpholinyl,
d) Phenyl,
e) Phenyl, ein- bis dreifach unabhängig voneinander substituiert durch
   1) (C₁-C₄)-Alkyl,
   2) (C₁-C₄)-Alkyl, ein- bis mehrfach substituiert durch Fluor-, Chlor-, Brom- oder Jodatom,
   3) Fluor-, Chlor-, Brom- oder Jodatom,
   4) Nitro,
   5) Aminosulfonyl,
   6) Amidino,
   7) Guanidino,
   8) (C₁-C₃)-Alkyloxy,
   9) Methylendioxy,
   10) Hydroxy,
   11) Carboxy,
   12) Cyano,
   13) (C₁-C₃)-Alkyoxycarbonyl,
   14) - CH = CH - COOH,
   15) 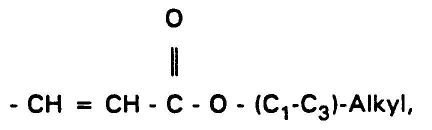
   16) Amino oder
   17) Amino, substituiert durch den Rest der Formel II, worin X und Y unabhängig voneinander
      17.1. Wasserstoffatom,
      17.2. (C₁-C₆)-Alkyl,
      17.3. (C₁-C₆)-Alkoxy,
      17.4. (C₃-C₆)-Alkenyloxy,
      17.5. Phenoxy oder
      17.6. Hydroxy bedeuten,
f) (C₅-C₇)-Cycloalkyl,
g) (C₅-C₇)-Cycloalkyl, ein- bis mehrfach unabhängig voneinander substituiert durch die unter c)1) bis c)4) genannten Reste,
h) Adamantyl,
i) (C₁-C₁₂)-Alkyl, substituiert durch Amino, unsubstituiert oder einbis zweimal substituiert durch (C₁-C₃)-Alkyl,
j) (C₁-C₁₂)-Alkyl, substituiert durch -C(O)-O-(C₁-C₃)-Alkyl,
k) Naphthyl,
l) Tetralin,
m) Pyridyl,
n) Pyridyl, ein- bis mehrfach unabhängig voneinander substiuiert wie unter e)1) bis e)17) definiert,
o) Pyrimidyl,
p) Pyrimidyl, ein- bis mehrfach unabhängig voneinander substituiert wie unter e)1) bis e)17) definiert, steht,
q) Piperidylrest, unsubstituiert oder ein- bis vierfach substituiert durch
   1) (C₁-C₄)-Alkyl,
   2) Phenyl oder
   3) Benzyl, steht, oder
r) R¹ und R² bilden zusammen mit dem Stickstoffatom, an das sie gebunden sind, einen Piperidinorest,
s) R¹ und R² bilden zusammen mit dem Stickstoffatom, an das sie gebunden sind, einen Piperazino- oder Morpholinorest, oder
t) R¹ und R² bilden zusammen mit dem Stickstoffatom, an das sie gebunden sind, einen Piperazinorest, der am Stickstoffatom substituiert ist durch
   1) Phenyl oder
   2) den Rest der Formel II, worin X und Y unabhängig voneinander die unter e)17.1. bis e)17.6. genannte Bedeutung haben, und
X und Y gleich oder verschieden sind und unabhängig voneinander die unter e)17.1. bis e)17.6. genannte Bedeutung haben;
und einen physiologisch verträglichen Träger.

Insbesondere bevorzugt ist ein Arzneimittel enthaltend mindestens eine Verbindung der Formel I und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₅)-Alkyl,
c) (C₁-C₅)-Alkyl, ein- bis mehrfach unabhängig voneinander substituiert durch
   1) Phenyl,
   2) Phenyl, ein- bis dreifach unabhängig voneinander substituiert durch
      2.1. Fluor oder Chloratom,
      2.2. Methyl, Ethyl oder Propyl,
      2.3. Methoxy,
      2.4. Methylendioxy,
      2.5. Nitro,
      2.6. Amino,
      2.7. Amidino,
      2.8. Guanidino,
      2.9. Hydroxy,
      2.10. Methoxycarbonyl oder
      2.11. Cyano, oder
   3) Morpholinyl,
d) (C₅-C₇)-Cycloalkyl,
e) (C₅-C₇)-Cycloalkyl, ein- bis dreifach unabhängig voneinander substituiert wie unter c)1) bis c)3) definiert,
f) Adamantyl,
g) Naphthyl,
h) Phenyl,
i) Phenyl, ein- bis dreifach unabhängig voneinander substituiert durch
   1) (C₁-C₂)-Alkyl,
   2) Trifluormethyl,
   3) Fluor- oder Chloratom,
   4) Nitro,
   5) Aminosulfonyl,
   6) Amidino,
   7) Guanidino,
   8) Methoxy,
   9) Methylendioxy,
   10) Hydroxy,
   11) Carboxy,
   12) Cyano,
   13) Methoxycarbonyl,
   14) - CH = CH - COOH,
   15) 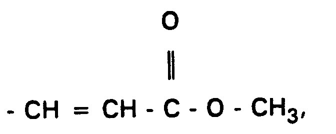
   16) Amino oder
   17) Amino, substituiert durch den Rest der Formel II, worin X und Y unabhängig voneinander
      17.1. Wasserstoffatom,
      17.2. Methoxy, Ethoxy, Propoxy oder Butyloxy
      17.3. Allyl,
      17.4. Phenoxy oder
      17.5. Hydroxy bedeuten,
k) Pyridyl,
l) Pyrimidyl steht, oder
m) R¹ und R² bilden zusammen mit dem Stickstoffatom, an das sie gebunden sind, einen Piperidinorest,
n) R¹ und R² bilden zusammen mit dem Stickstoffatom, an das sie gebunden sind, einen Piperazino- oder Morpholinorest, oder
o) R¹ und R² bilden zusammen mit dem Stickstoffatom, an das sie gebunden sind, einen Piperazinorest, der am Stickstoffatom substituiert ist durch
   1) Phenyl oder
   2) den Rest der Formel II, worin X und Y unabhängig voneinander die unter i)17.1. bis i)17.5. genannte Bedeutung haben, und
X und Y gleich oder verschieden sind und unabhängig voneinander die unter i)17.1. bis i)17.6. genannte Bedeutung haben;
und einen physiologisch verträglichen Träger.

Ferner ist bevorzugt ein Arzneimittel, enthaltend mindestens eine Verbindung der Formel I und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) Phenyl,
c) Phenyl, ein- bis dreifach unabhängig voneinander substituiert durch
   1) Fluor- oder Chloratom,
   2) Methylendioxy oder
   3) (C₁-C₃)-Alkoxy, steht und
X und Y unabhängig voneinander
1) Wasserstoffatom,
2) (C₁-C₃)-Alkoxy oder
3) Hydroxy, bedeutet;
und ein physiologisch verträglicher Träger.

Unter dem Begriff Alkyl oder Alkoxy werden Reste verstanden deren Kohlenstoffkette geradkettig, verzweigt oder cyclisch sein kann. Cyclische Alkylreste sind beispielsweise 5- bis 7-gliedrige Monocyclen wie Cyclopentyl, Cyclohexyl oder Cycloheptyl. Ferner gehören zu den cyclischen Alkylresten auch Polycyclen wie Adamantan-, Twistan- oder Diamantanreste.

Zu dem Begriff "monocyclischer 5- oder 6-gliedriger gesättigter, heterocyclischer Ring" gehören beispielsweise Reste, die sich von Pyrrolidin, Piperidin, Pyrazolidin, Imidazolidin, Piperazin, Isoxazolidin, Morpholin, Isothiazolidin oder Thiomorpholin ableiten.

Zu dem Begriff "R¹ und R² bilden zusammen mit dem Stickstoffatom, an das sie gebunden sind, einen 4- bis 7-gliedriger Ring" gehören beispielsweise Reste, die sich von Azetidin, Pyrrolidin, Piperidin oder Azepin ableiten.

Zu dem Begriff "R¹ und R² bilden zusammen mit dem Stickstoffatom, an das sie gebunden sind, einen 5- bis 7-gliedrigen Ring, worin ein ringständiges C-Atom durch eine Sauerstoffatom oder Stickstoffatom ersetzt ist" gehören beispielsweise Reste die sich von Isoxazolidin, Morpholin, Imidazolidin oder Piperazin ableiten.

Der Begriff "Heteroaryl" steht für aromatische Ringsysteme, die außer Kohlenstoff- noch Heteroatome als Ringglieder enthalten, z. B. Stickstoff, Sauerstoff oder Schwefel. Bevorzugt sind 5- oder 6-gliedrige monocyclische Reste wie Pyrrol, Pyridin, Furan, Thiophen, Pyrazol, Imidazol, Pyridazin, Pyrimidin, Pyrazin, Oxazol, Isoxazol, Thiazol oder Isothiazol. Ferner sind bevorzugt 9- bis 10-gliedrige bicyclische Ringe wie Indol, Chinon, Isochinolin, Indazol, Benzimidazol, Phthalazin, Chinazolin, Chinoxalin, Purin oder Pteridin.

Degenerative Gelenkserkrankungen sind beispielsweise Arthrosen, Erkrankungen des rheumatischen Formenkreises mit Knorpelabbau, chronischer Polyarthritis, Knorpelschwund nach Gelenktrauma wie nach Meniskus- oder Pattellaverletzungen oder Bänderrissen, oder Knorpelschwund nach Stillegung von Gelenken.

Die Erfindung betrifft ferner neue Verbindungen der Formel I und/oder physiologisch verträgliche Salze der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₁₂)-Alkyl, ein- bis mehrfach unabhängig voneinander substituiert durch
   1) Phenyl, ein- bis dreifach unabhängig voneinander substituiert durch
      1.1. Fluor-, Chlor-, Brom- oder Jodatom,
      1.2. (C₁-C₃)-Alkyl,
      1.3. (C₁-C₃)-Alkoxy,
      1.4. Methylendioxy,
      1.5. Nitro,
      1.6. Amino,
      1.7. Amidino,
      1.8. Guanidino,
      1.9. Hydroxy,
      1.10. (C₁-C₆)-Alkoxycarbonyl oder
      1.11. Cyano,
   2) einen monocyclischen 5- oder 6-gliedrigen gesättigten heterocyclischen Ring,
   3) Amino, unsubstituiert oder ein- bis zweimal substituiert durch (C₁-C₃)-Alkyl, oder
   4) -C(O)-O-(C₁-C₃)-Alkyl,
c) Tetralin,
d) Phenyl, ein- bis dreifach unabhängig voneinander substituiert durch
   1) (C₁-C₄)-Alkyl,
   2) (C₁-C₄)-Alkyl, ein- bis mehrfach substituiert durch Fluor-, Chlor-, Brom- oder Jodatom,
   3) Nitro,
   4) Aminosulfonyl,
   5) Amidino,
   6) Guanidino,
   7) (C₁-C₃)-Alkyloxy,
   8) Methylendioxy,
   9) Hydroxy,
   10) Carboxy,
   11) Cyano,
   12) (C₁-C₃)-Alkyoxycarbonyl,
   13) - CH = CH- COOH,
   14) 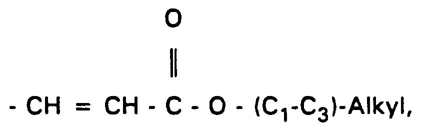
   15) Amino oder
   16) Amino, substituiert durch den Rest der Formel II, worin X und Y unabhängig voneinander
      16.1. Wasserstoffatom,
      16.2. (C₁-C₆)-Alkyl,
      16.3. (C₁-C₆)-Alkoxy,
      16.4. (C₃-C₆)-Alkenyloxy,
      16.5. Phenoxy oder
      16.6. Hydroxy bedeuten,
e) Naphthyl, ein- bis mehrfach substituiert wie unter d)1) bis d)16) definiert,
f) Heteroaryl,
g) Heteroaryl, ein- bis mehrfach substituiert durch Phenyl, Benzyl oder wie unter d)1) bis d)16) definiert, steht, oder
h) R¹ und R² bilden zusammen mit dem Stickstoffatom, an das sie gebunden sind, einen 5- bis 7-gliedrigen Ring, worin ein ringständiges C-Atom durch ein Sauerstoffatom oder ein Stickstoffatom ersetzt ist, oder
i) R¹ und R² bilden zusammen mit dem Stickstoffatom, an das sie gebunden sind, einen 5- bis 7-gliedrigen Ring, worin ein ringständiges C-Atom durch ein Stickstoffatom ersetzt ist und dieses Stickstoffatom ist substituiert durch
   1) Phenyl oder
   2) den Rest der Formel II, worin X und Y unabhängig voneinander die unter d)16.1. bis d)16.6. genannte Bedeutung haben, und
X und Y gleich oder verschieden sind und unabhängig voneinander die unter d)16.1. bis d)16.6. genannte Bedeutung haben; oder wobei
- R¹ für: Phenyl oder Phenyl, ein- bis dreifach substituiert durch Fluor-, Chlor-, Brom- oder Jodatom, steht
R² für Wasserstoffatom steht und
X und Y unabhängig voneinander für
1) Wasserstoffatom,
2) (C₁-C₆)-Alkyl,
3) (C₃-C₆)-Alkenyloxy,
4) Phenoxy oder
5) Hydroxy stehen.

Bevorzugt sind neue Verbindungen der Formel I , wobei
R¹ und R² gleich oder verschieden sind und unabhängig voneinander für
a) Wasserstoffatom,
b) (C₁-C₁₂)-Alkyl, ein- bis mehrfach unabhängig voneinander substituiert durch
   1) Phenyl, ein- bis dreifach unabhängig voneinander substituiert durch
      1.1. Fluor-, Chlor-, Brom- oder Jodatom
      1.2. Methylendioxy oder
      1.3. Cyano,
   2) Amino, unsubstituiert oder ein- bis zweimal substituiert durch (C₁-C₃)-Alkyl, oder
   3) -C(O)-O-(C₁-C₃)-Alkyl,
c) Phenyl, ein- bis dreifach unabhängig voneinander substituiert durch
   1) (C₁-C₄)-Alkyl, ein- bis mehrfach substituiert durch Fluor-, Chlor-, Brom- oder Jodatom,
   2) (C₁-C₃)-Alkyloxy,
   3) Methylendioxy,
   4) Cyano oder
   5) Amino, substituiert durch den Rest der Formel II, worin X und Y unabhängig voneinander
      5.1. Wasserstoffatom,
      5.2. (C₁-C₆)-Alkyl,
      5.3. (C₁-C₆)-Alkoxy,
      5.4. (C₃-C₆)-Alkenyloxy,
      5.5. Phenoxy oder
      5.6. Hydroxy bedeuten,
d) Pyridyl oder
e) Pyrimidyl steht, und worin
X und Y unabhängig voneinander die unter c)5.1 bis c)5.6 genannte Bedeutung haben; oder wobei
- R¹ für: Phenyl oder Phenyl, ein- bis dreifach substituiert durch Fluor- oder Chloratom, steht,
- R² für: Wasserstoffatom steht und
X und Y unabhängig voneinander für
1) Wasserstoffatom,
2) (C₁-C₂)-Alkyl oder
3) Hydroxy stehen.

Die Erfindung betrifft weiterhin Verfahren zur Herstellung der Verbindung der Formel I, wobei eine Ausführungsform darin besteht, daß man
A) einen Phosphorigsäurediester oder Phosphorigsäuremonoester der Formel III, 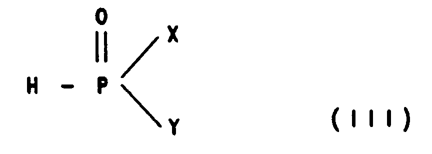 wobei X und Y unabhängig voneinander für
   a) Wasserstoffatom,
   b) (C₁-C₆)-Alkyl,
   c) (C₁-C₆)-Alkoxy,
   d) (C₃-C₆)-Alkenyloxy oder
   e) Phenoxy stehen,
   mit einem Azomethin der Formel IV, 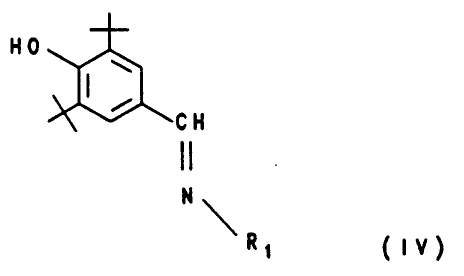 wobei R¹ die unter der Formel I für b) bis k) genannte Bedeutung hat, zu einer Verbindung der Formel Ia umsetzt 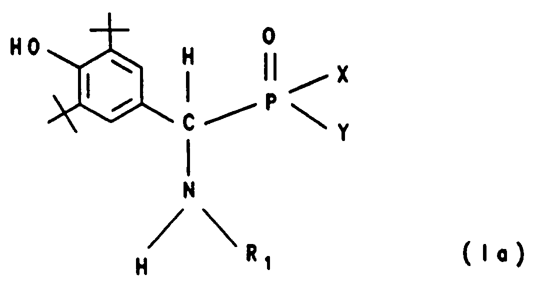 wobei R¹ die in Formel IV sowie X und Y die in Formel III angegebene Bedeutung haben, oder
B) eine Verbindung der Formel NHR¹R²,
   wobei R¹ und R² unabhängig voneinander die in Formel I angegebene Bedeutung haben,
   in Gegenwart von 4-Hydroxy-3,5-di-tert.butylbenzaldehyd und einer Verbindung der Formel III zu einer Verbindung der Formel I umsetzt, oder
C) eine Additionsverbindung der Formel V 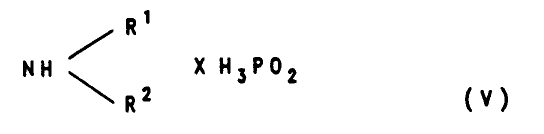 wobei R¹ und R² unabhängig voneinander die in Formel I angegebene Bedeutung haben,
   in Gegenwart von 4-Hydroxy-3,5-di-tert.butylbenzaldehyd zu einer Verbindung der Formel Ib 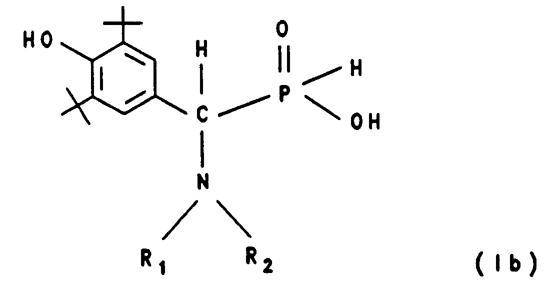 umsetzt, oder
D) eine Verbindung der Formel I, wobei X und Y unabhängig voneinander für
   a) (C₁-C₆)-Alkyl,
   b) (C₁-C₆)-Alkoxy,
   c) (C₃-C₆)-Alkenyloxy oder
   d) Phenoxy, stehen,
   zu einer Verbindung der Formel I, wobei X und Y unabhängig voneinander für Hydroxy stehen, hydrolysiert, oder
E) eine nach Verfahren A) - D) hergestellte Verbindung der Formel I, die aufgrund ihrer chemischen Struktur in enantiomenren Formen auftritt, durch Salzbildung mit enantiomerenreinen Säuren oder Basen, Chromatographie an chiralen Stationärphasen oder Derivatisierung mittels chiraler enantiomerenreiner Verbindungen wie Aminosäuren, Trennung der somit erhaltenen Diastereomeren, und Abspaltung der chiralen Hilfsgruppe in die reinen Enantiomeren auftrennt, oder
F) die nach Verfahren A) - E) hergestellte Verbindung der Formel I entweder in freier Form isoliert oder im Falle des Vorliegens von sauren oder basischen Gruppen gegebenenfalls in physiologisch verträgliche kristalline Salze umwandelt.

Bei der Verfahrensvariante A) geht man beispielsweise so vor, daß man die Verbindung der Formel III an die C = N-Doppelbindung der Azomethine - Verbindung der Formel IV - addiert (E. K. Fields, J. Am. Chem. Soc. 74 (1952) 1528; K. Issleib et al. Z. Naturforsch. 36 b (1981) 1392). Bei der Umsetzung werden vorzugsweise äquimolare Mengen der Verbindung der Formel III und Formel IV eingesetzt. Die Reaktion erfolgt in Gegenwart von unpolaren Lösungsmitteln wie Toluol, Xylol, Benzol, Cyclohexan oder höhersiedenden Kohlenwasserstoffen aber auch in polaren Lösemitteln wie Alkoholen, z. B. Methanol, Ethanol, Propanol oder Butanol aber auch in niederen aliphatischen Carbonsäuren wie Essigsäure, Dimethylformamid und Acetonitril oder Mischungen der genannten Lösemitteln. Die Reaktion läßt sich auch ohne Zusatz von Lösemitteln durchführen, insbesondere dann, wenn als Phosphor-Komponente Phosphorigsäurediester wie Phosphorigsäurediethylester in äquimolaren Mengen oder in bis zu 10-fachem Überschuß eingesetzt wird.

Die Reaktion wird im Bereich von Raumtemperatur bis zur Siedetemperatur des jeweils verwendeten Lösemittels durchgeführt, bevorzugt bei 60° bis 120° C, die Reaktionszeiten betragen 2 bis 6 Stunden. Eine bevorzugte Variante dieses Verfahrens besteht in der Verwendung einer Lewissäure wie Aluminiumchlorid, die in bis zu äquimolaren Mengen zugegeben wird, wobei vorzugsweise die Reaktion in einem inerten Lösemittel wie Toluol, zweckmäßigerweise bei Siedetemperatur des Lösemitteln erfolgt.
Die für das Verfahren notwendigen Azomethine der Formel IV lassen sich nach dem Fachmann bekannten Verfahren herstellen, z. B. durch Kondensation von 4-Hydroxy-3,5-di-tert.butylbenzaldehyd mit den entsprechenden primären Aminen H₂N-R₁ in Gegenwart eines Katalysators, beispielsweise p-Toluolsulfonsäure in einem bevorzugt unpolaren Lösemittel wie Toluol oder Benzol unter Abscheidung des freigesetzten Wassers.

Bei der Verfahrensvariante B) geht man beispielsweise so vor, daß man die Reaktionspartner - Verbindung der Formel NHR¹R², 4-Hydroxy-3,5-di-tert.butylbenzaldehyd und die Verbindung der Formel III - in äquimolaren Mengen umsetzt. Die Reaktion erfolgt in einem mit Wasser nicht mischbaren Lösemittel wie Toluol oder Benzol. Als besonders vorteilhaft erweist sich zur Beschleunigung der Wasserabspaltung die Zugabe einer Lewissäure, wie AlCl₃, ZnCl₂ oder Sulfonsäuren wie p-Toluolsulfonsäure. Bevorzugte Reaktionstemperatur ist von etwa 50 °C bis zur Siedetemperatur des Lösemittels, wobei die Reaktionszeiten 2 bis 6 Stunden betragen.

Die Reaktionsbedingungen der Verfahrensvariante C) sind die gleichen wie bei den Verfahrenvarianten A) und B). Die Herstellung der Verbindung der Formel V erfolgt durch Umsetzung äquimolarer Mengen von Amin und hypophosphoriger Säure in einem inerten Lösemittel wie Acetonitril, einem Ether, z. B. Diethylether oder tert.Butylmethylether, Toluol oder Methylenchlorid. Die Umsetzung erfolgt bei Temperaturen von 20° bis 50° C.

Die Hydrolyse gemäß Verfahrensvariante D) erfolgt nach bekannten Verfahren. Vorzugsweise erfolgt die Hydrolyse durch acidolytische Spaltung mit Bromwasserstoffsäure in Eisessig, bevorzugt bei Raumtemperatur und Reaktionszeiten bis zu 8 Stunden.

Die Herstellung physiologisch verträglicher Salze aus zur Salzbildung befähigten Verbindungen der Formel I, einschließlich deren stereoisomeren Formen, erfolgt in an sich bekannter Weise. So bilden die Carbonsäuren, Phosphon- und Phosphinsäuren sowie die Phosphonsäurehalbester mit basischen Reagenzien, wie Hydroxiden, Carbonaten, Hydrogencarbonaten, Alkoholaten sowie Ammoniak oder organischen Basen, beispielsweise Trimethyl- oder Triethylamin, Ethanolamin oder auch basischen Aminosäuren, etwa Lysin, Ornithin oder Arginin stabile Alkali-, Erdalkali- oder gegebenenfalls substituierte Ammoniumsalze. Sofern die Verbindungen der Formel I basische Gruppen im Rest R¹ oder R² aufweisen, lassen sich mit starken Säuren auch stabile nichttoxische Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, oder Trifluoressigsäure in Frage.

Sofern die Verbindungen der Formel I in diastereoisomeren oder enantiomeren Formen auftreten und bei der gewählten Synthese als deren Gemische anfallen, gelingt die Trennung in die reinen Stereoisomeren entweder durch Chromatographie an einem gegebenenfalls chiralen Trägermaterial, oder, sofern die racemischen Verbindungen der Formel I zur Salzbildung befähigt sind, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren, Phosphonsäuren, und Phosphinsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Die Erfindung betrifft ferner die Verwendung der Verbindung der Formel I zur Herstellung von Arzneimitteln zur Prophylaxe und Therapie von degenerativen Gelenkserkrankungen.

Die Erfindung betrifft auch ein Verfahren zur Herstellung eines Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Die erfindungsgemäßen Arzneimittel werden oral, intramuskulär, periartikulär, intraartikulär, intravenös, intraperitoneal, subkutan oder rektal verabreicht.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole, z. B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

### Beispiel 1

### nach Verfahrensvariante A)

### 1-(4-Chloranilino)-1-(3,5-di-tert.butyl-4-hydroxy-phenyl)-methanphosphonsäurediethylester

### a1) N-(3,5-di-tert.butyl-4-hydroxybenzyliden-4-chloranilin

12,2 g (50 mmol) 3,5-Di-tert.butyl-4-hydroxybenzaldehyd-semihydrat und 6,4 g (50 mmol) 4-Chloranilin in 130 ml Toluol werden zusammen mit 0,2 g p-Toluolsulfonsäure unter Rückfluß erhitzt, bis sich die berechnete Menge an Reaktionswasser azeotrop abgeschieden hat. Nach Einengen des Reaktionsgemisches unter vermindertem Druck wird der Rückstand aus Ethanol umkristallisiert.
Ausbeute: 15,0 g (87 % der Theorie)
Schmelzpunkt: 108 - 110° C

### a2) 1-(4-Chloranilino-1-(3,5-di-tert.butyl-4-hydroxy-phenyl)methanphosphonsäurediethylester

Eine Lösung von 15,0 g (44 mmol) N-(3,5-di-tert.butyl-4-hydroxybenzyliden-4-chloranilin und 7,0 g (50 mmol) Phosphorigsäurediethylester in 100 ml Toluol wird in Gegenwart von 0,1 g wasserfreiem AlCl₃ unter gleichzeitiger azeotroper Entfernung von Wasser 6 Stunden unter Rühren am Rückfluß erhitzt. Der nach dem Einengen des Reaktionsgemisches verbleibende Rückstand wird aus Diisopropylether umkristallisiert.
Ausbeute: 19,5 g (92 % der Theorie)
Schmelzpunkt: 130 - 131° C
C₂₅H₃₅ClNO₄P (MG = 482,0)

| Analyse: | | | | | |
|---|---|---|---|---|---|
| berechnet: | C 62,30 % | H 7,74 % | Cl 7,36 % | N 2,81 % | P 6,43 % |
| gefunden: | C 61,98 % | H 7,73 % | Cl 7,47 % | N 2,97 % | P 6,70 % |

### Beispiel 2

### nach Verfahrenvariante B)

### 1-(3,4-Methylendioxybenzylamino)-1-(3,5-di-tert.butyl-4-hydroxy-phenyl)methanphosphonsäure-di-n-propylester-hydrochlorid

Zu einer Lösung von 12,2 g (50 mmol) 3,5-Di-tert.butyl-4-hydroxybenzaldehydsemihydrat und 7,5 g (50 mmol) 3,4-Methylendioxybenzylamin sowie 300 mg p-Toluolsulfonsäure in 100 ml Toluol werden nach dem Erhitzen auf Rückflußtemperatur 9,1 g (50 mmol) Phosphorigsäuredipropylester und 0,1 g wasserfreies Aluminium-III-chlorid unter Rühren zugegeben. Nach 6-stündigem Erhitzen unter Rückfluß unter gleichzeitiger azeotroper Entfernung des Reaktionswassers, wird das Gemisch heiß filtriert und nach dem Abkühlen mit methanolischer Salzsäure versetzt. Die in Form des Hydrochlorides ausfallende Substanz wird anschließend aus Acetonitril umkristallisiert.
Ausbeute: 18,1 g (63 % der Theorie)
Schmelzpunkt: 162 - 163° C
C₂₉H₄₅ClNO₆P (MG = 570,11)

| Analyse: | | | | |
|---|---|---|---|---|
| berechnet: | C 61,10 % | H 7,96 % | N 2,46 % | P 5,43 % |
| gefunden: | C 59,80 % | H 8,30 % | N 2,45 % | P 4,93 % |

### Beispiel 3

### nach Verfahrensvariante C)

### 1-(3,4-Methylendioxybenzylamino)-1-(3,5-di-tert.butyl-4-hydroxy-phenyl)methanphosphinsäure

### c1) 3,4-Methylendioxyanilin-hypophosphit

Zu einer Lösung von 13,8 g (0,1 mol) 3,4-Methylendioxyanilin in 20 ml Acetonitril werden unter Rühren bei Raumtemperatur 13,2 g (0,12 mol) 60 %ige hypophosphorige Säure langsam zugetropft. Der sich nach 2-stündigem Rühren und Stehenlassen über Nacht bildende Niederschlag wird abfiltriert, mit Acetonitril nachgewaschen und unter verminderten Druck getrocknet.
Ausbeute: 14,0 g (69 % der Theorie)
Schmelzpunkt: 132 - 136° C
C₇H₁₀NO₄P (MG = 203,14)

### c2) 1-(3,4-Methylendioxybenzylamino)-1-(3,5-dit-tert.butyl-4-hydroxy-phenyl)methanphosphinsäure

Ein Gemisch von 11,7 g (50 mmol) 3,5-Di-tert.butyl-4-hydroxybenzaldehyd und 10,15 g (50 mmol) 3,4-Methylendioxyanilin-hypophosphit in 400 ml Acetonitril wird unter Rühren 4 Stunden unter Rückfluß erhitzt. Nach dem Abkühlen wird der ausgefallene Niederschlag abfiltriert und mehrmals mit heißem Aceton digeriert.
Ausbeute: 19,0 g (91 % der Theorie)
Schmelzpunkt: 196 - 197° C
C₂₂H₃₀NO₅P (MG = 419,46)

| Analyse: | | | | |
|---|---|---|---|---|
| berechnet: | C 63,00 % | H 7,21 % | N 3,34 % | P 7,38 % |
| gefunden: | C 62,72 % | H 6,97 % | N 3,29 % | P 7,26 % |

### Beispiel 4

### nach Verfahren D)

### 1-(4-Chloranilino-1-(3,5-di-tert.butyl-4-hydroxyphenyl)-methan-phosphonsäure

Zu 2 g (4,4 mmol) 1-(4-Chloranilino-1-(3,5-di-tert.butyl-4-hydroxyphenyl)methanphosphonsäuredimethylester werden 20 ml 33 % Bromwasserstoffsäure in Eisessig zugegeben und anschließend 1,5 Stunden bei Raumtemperatur gerührt. Die mit Wasser versetzte Mischung wird mit Essigester extrahiert, mit Wasser gewaschen und die organische Phase über Na₂SO₄ getrocknet. Nach dem Einengen erfolgt die Umkristallisation aus i-Propanol/Diisopropylether.
Ausbeute: 1,45 g (78 % der Theorie)
Schmelzpunkt: 162 - 165° C
C₂₁H₂₉ClNO₄P (MG = 425,9).

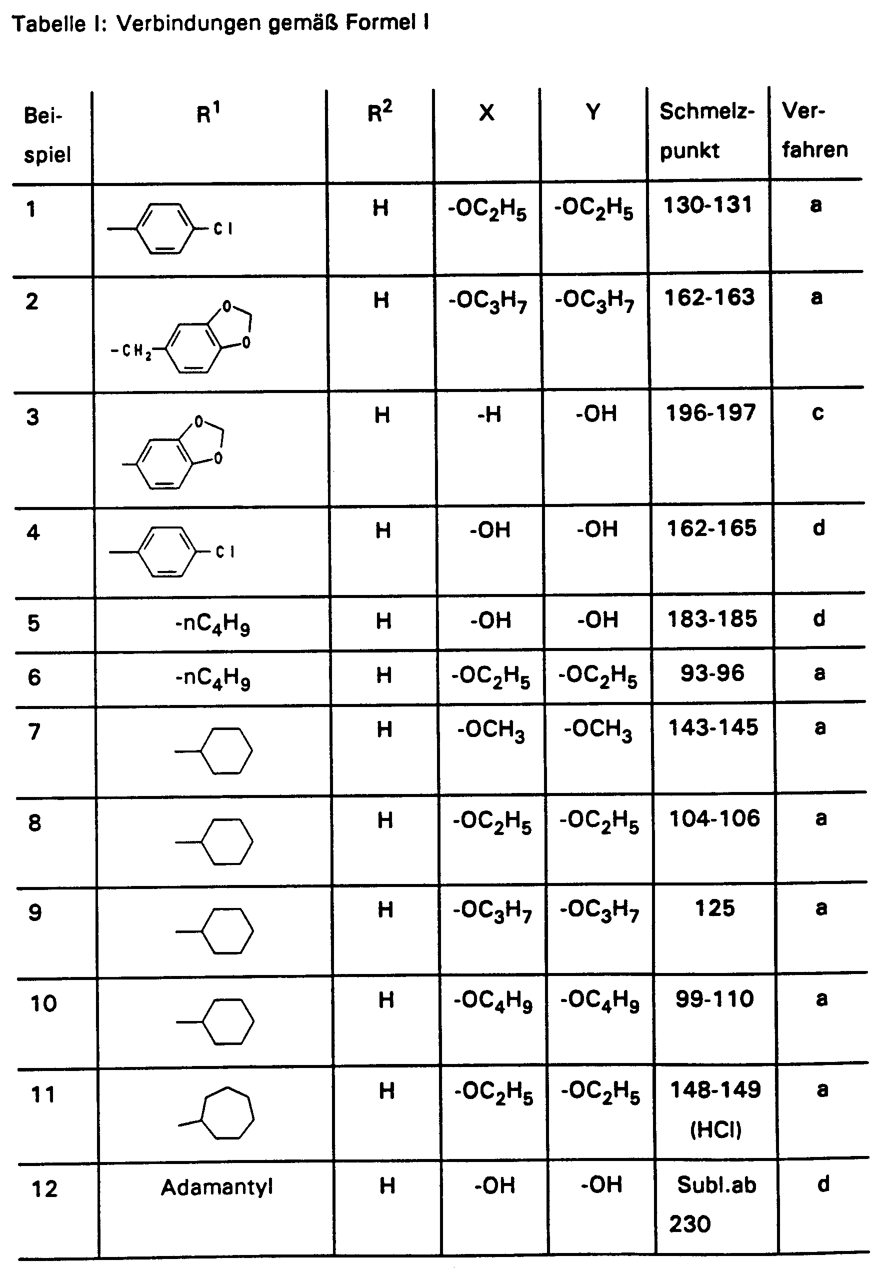

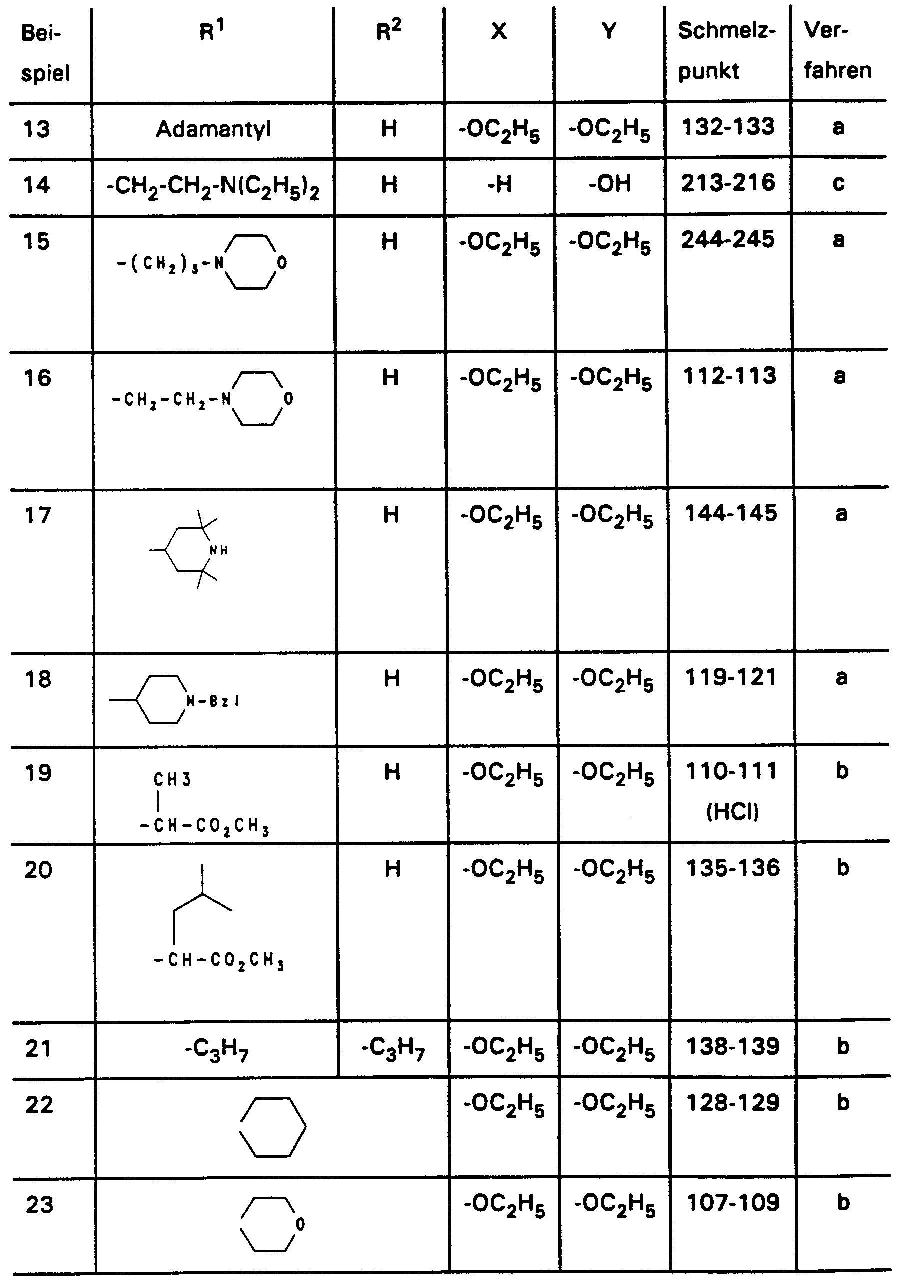

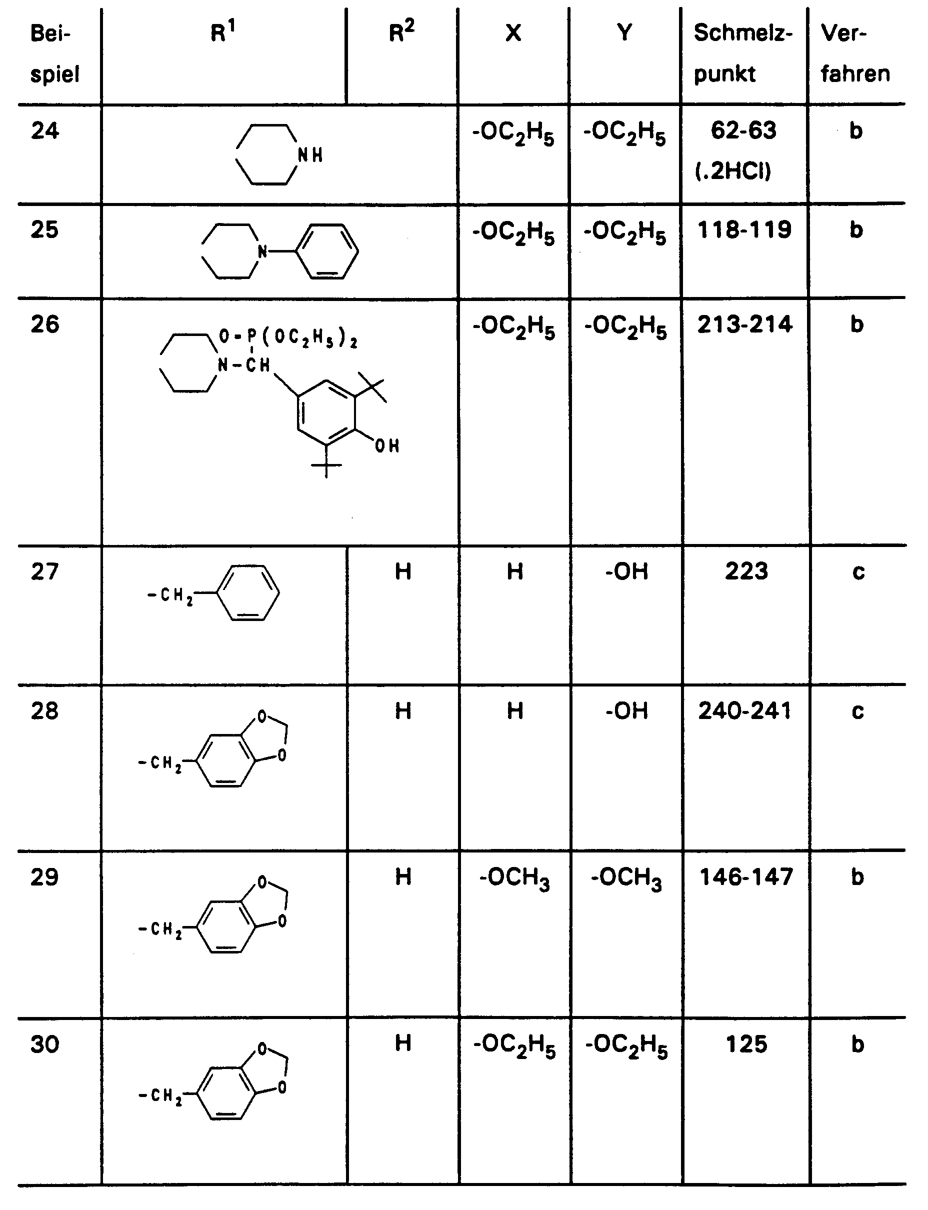

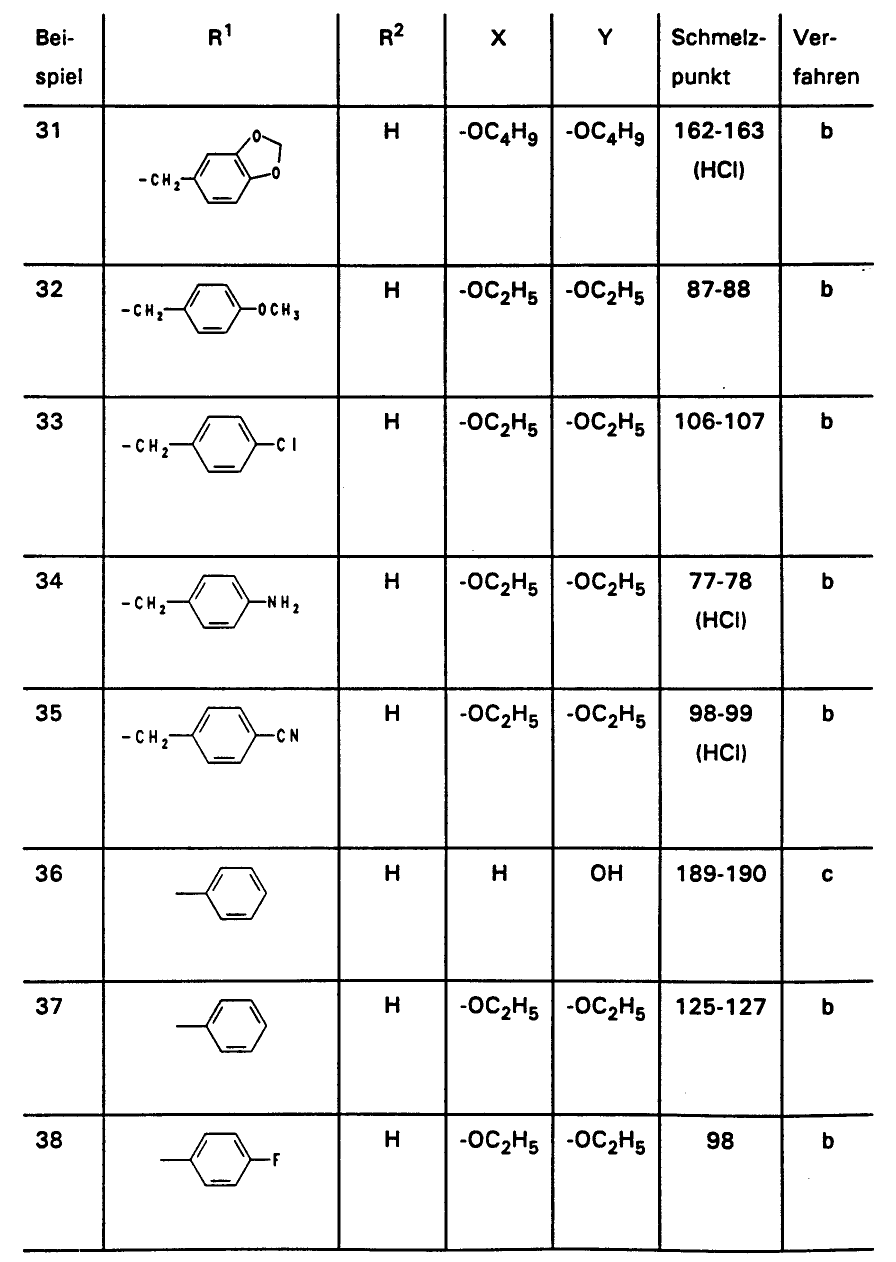

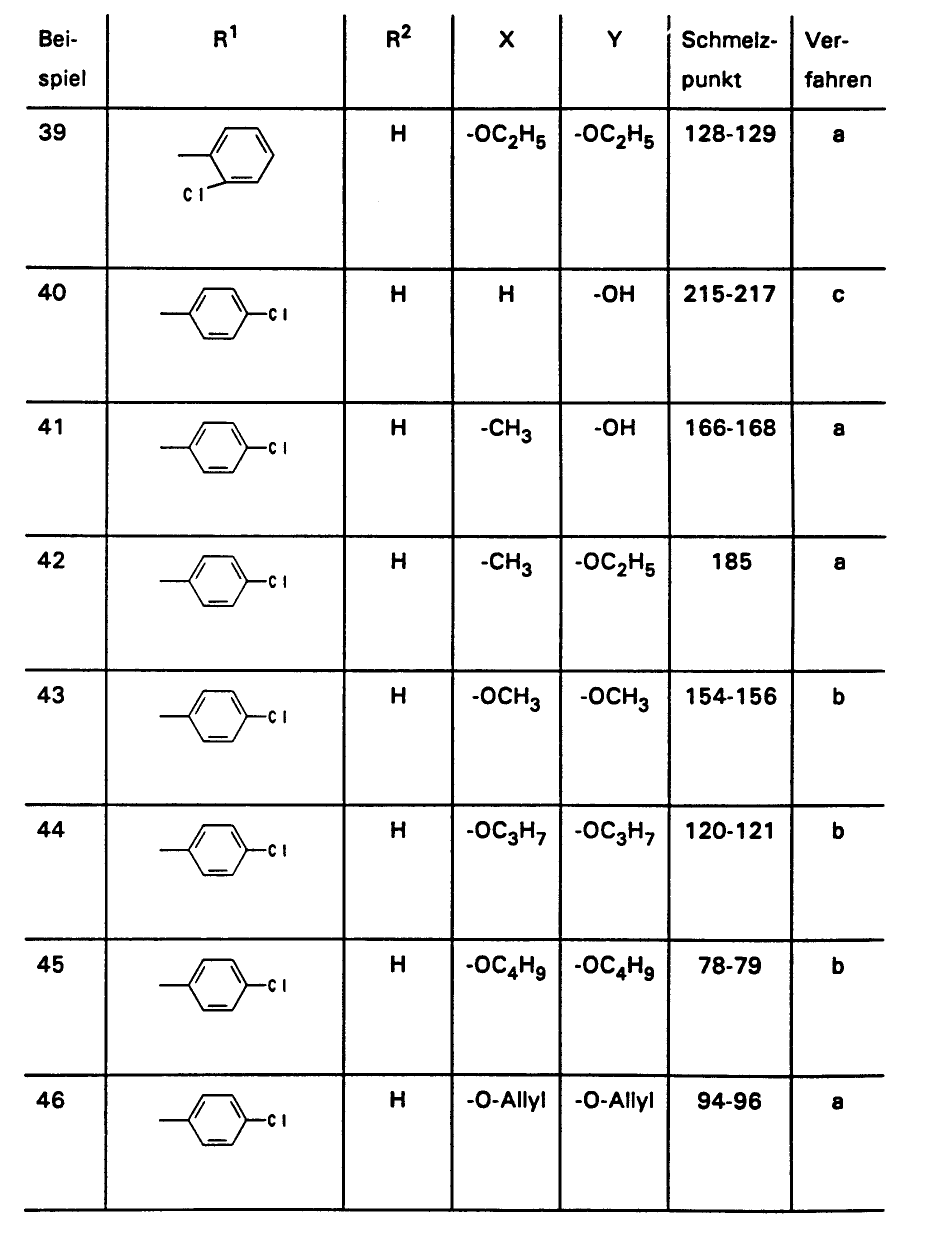

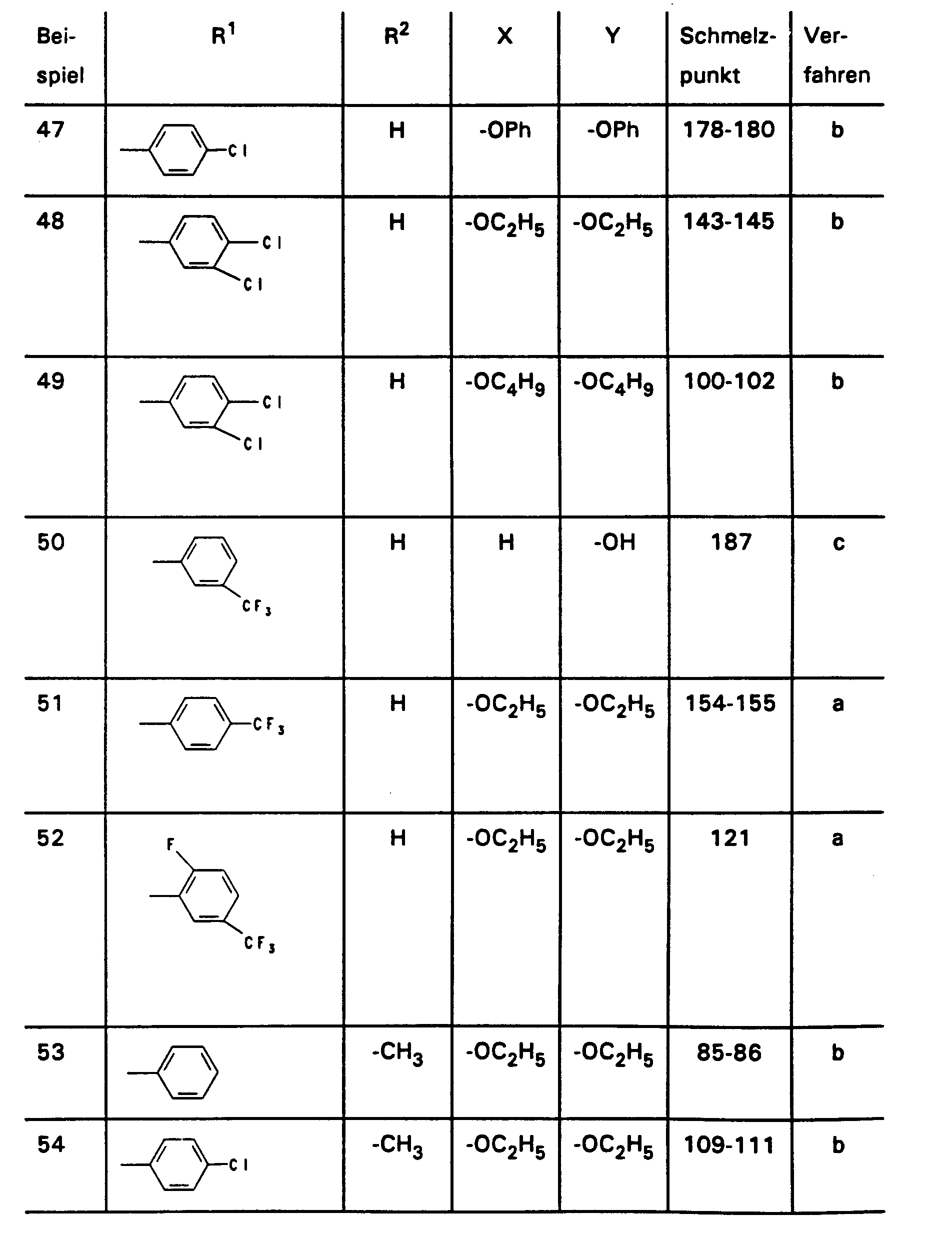

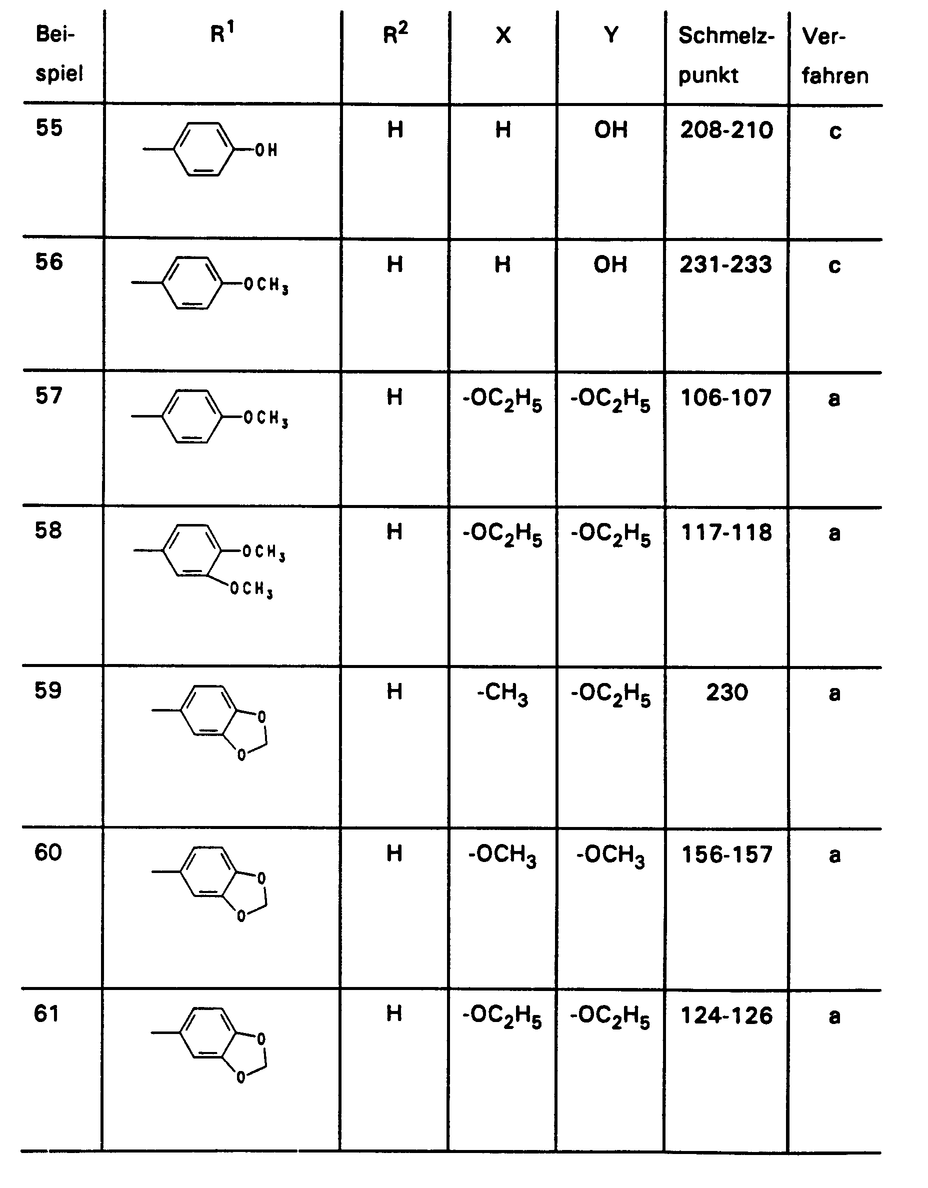

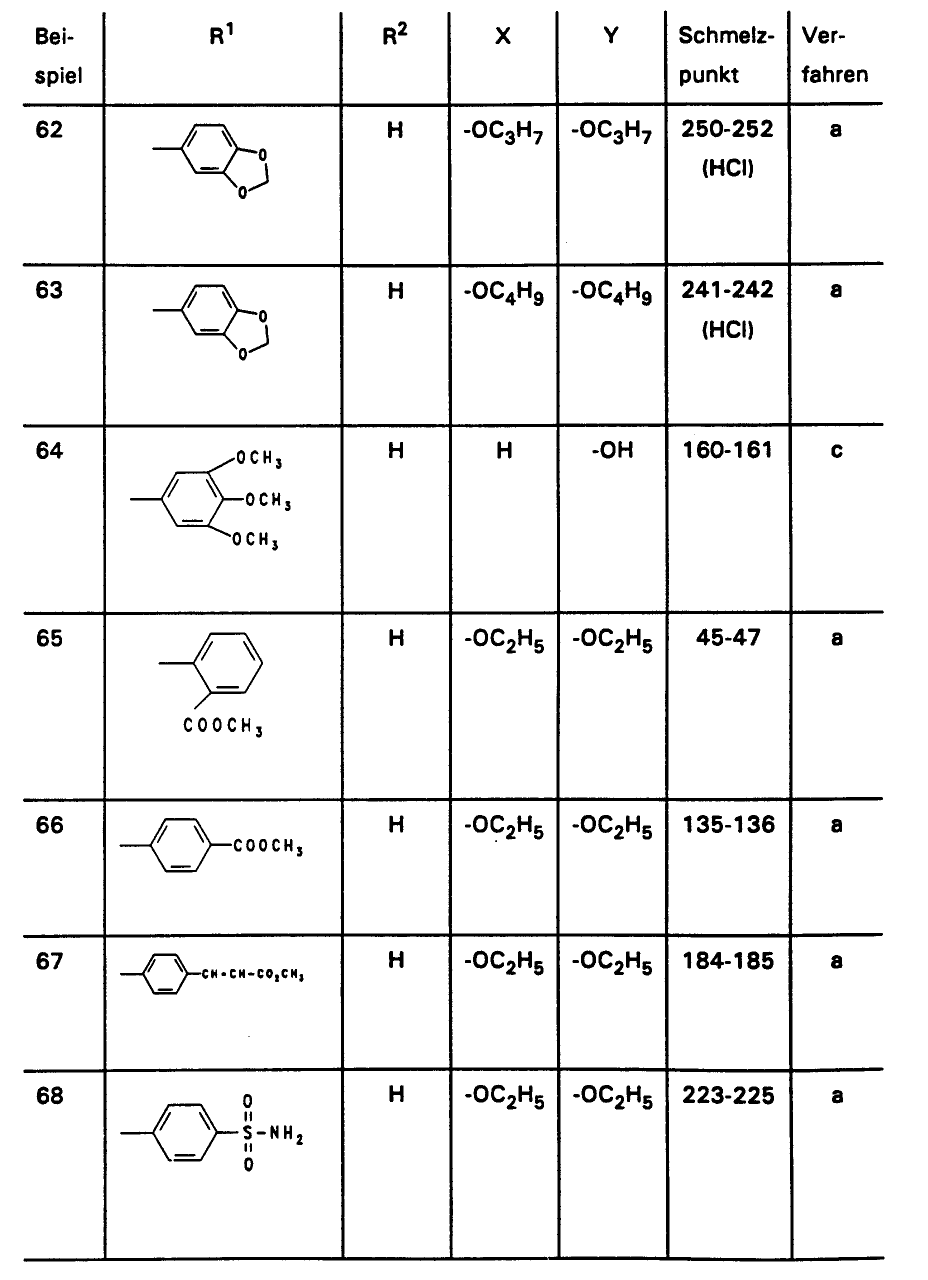

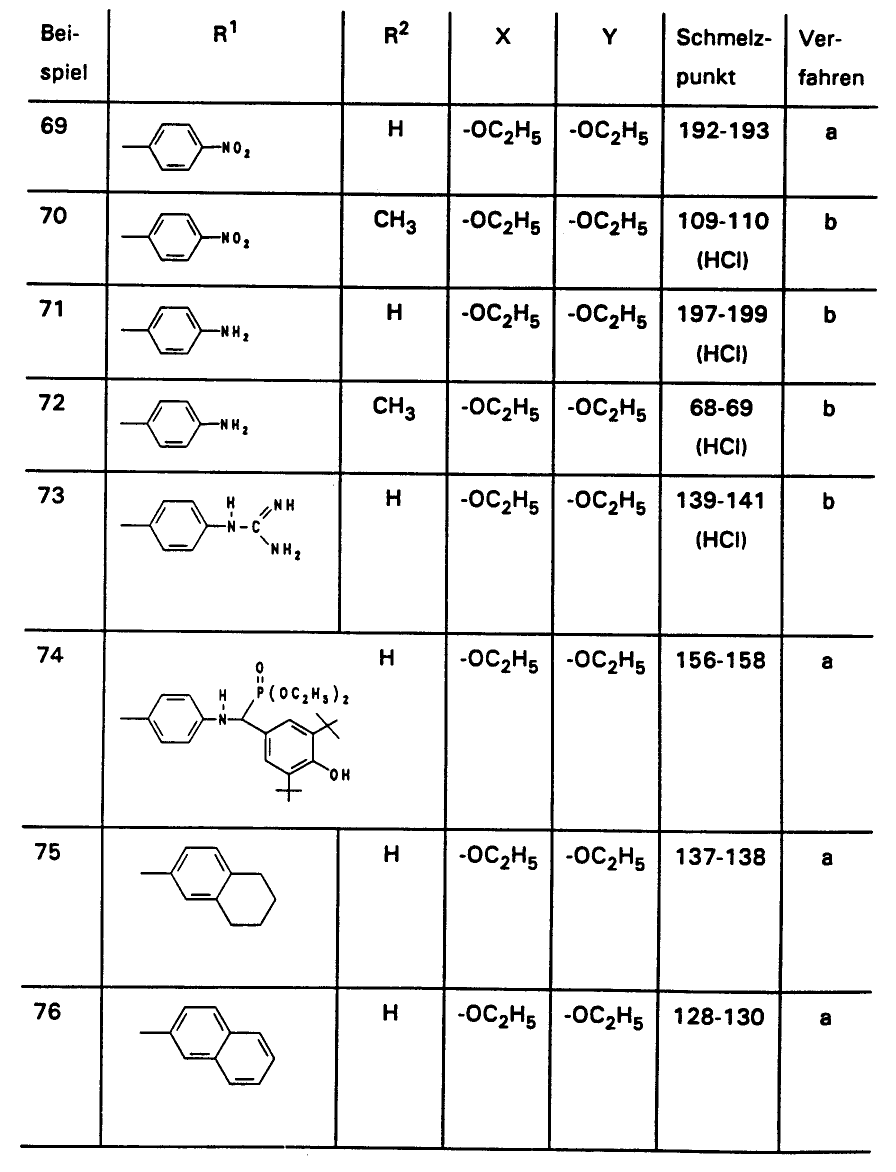

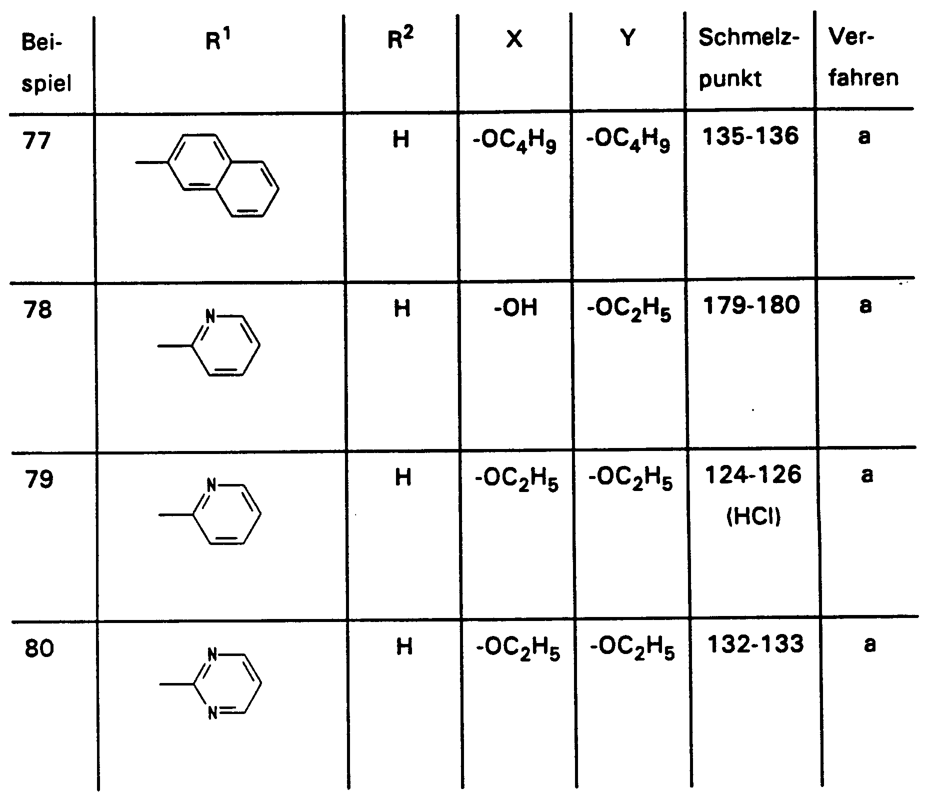

### Pharmakologische Prüfungen

### 1. Wirksamkeit in der Synthese-Stimulierung von Knorpel-Matrix, Prüfung in der Chondrozyten-Kultur

Zellen: Den Fußgelenken frisch geschlachteter Rinder werden der hyaline Knorpel entnommen, mit Pronase (Boehringer Mannheim) und Collagenase (Sigma), die native Matrix enzymatisch abgebaut, und die Chondrozyten in 1 %iger "low melting Agarose" in 24er Multiwell-Schalen in einer Zelldichte von 4 x 10⁶ pro well ausplattiert.

Medium: Komplettes Medium enthält HAM's F12 (Biochrom KG, Berlin) und 10 % foetales Kälberserum (Boehringer Mannheim), die Testsubstanz wird in Medium gelöst, üblicherweise in einer Konzentration von 10⁻⁵ M zugegeben und bei jedem Mediumwechsel erneut zugefügt.

Versuchsdurchführung: Die Behandlung erfolgt vom dritten bis zehnten Tag der Primärkultur, am 9. Tag wird 20 µCi/ml (7,4 x 10⁵ Bq) Na₂³⁵SO₄ für 24 h dem Medium zugefügt. Die dissoziative Extraktion der Proteoglykane aus der Agarose-Schicht wird mit 8M Guanidinium-Hydrochlorid und beigefügten Proteinase-Inhibitoren (Sigma) unter Schütteln bei 4° C über 24 h durchgeführt. Der Überstand wird nach Zentrifugation über eine PD 10 ®Sephadex G 25 Säule in freies und inkorporiertes Sulfat getrennt, dessen Aktivität nach Aliquotierung im β-Szintillationscounter gemessen wird.

Auswertung: Der Parameter für die Matrixproduktion der Chondrozyten ist die Menge synthetisierter Proteoglykane, gemessen als Sulfatinkorporation in cpm. Aus vier Wells pro Gruppe wird der Mittelwert errechnet. Dieser wird durch den Mittelwert der unbehandelten Kontrolle dividiert, und ergibt somit einen Stimulationsfaktor, der bei Stimulation der Matrixsynthese größer 1 ist, bei Hemmung derselben durch Substanzwirkung kleiner 1, und bei unveränderter Matrixsynthese gleich 1 ist.

Als Standard wird Diacerein eingesetzt, das unter dem Markennamen "Artodar" von der Firma Proter bzw. Fisiodar der Firma Gentili in Italien als Therapeutikum für Arthrose im Einsatz ist (Drugs of the Future 11 (6), 1986).

**Tabelle II**

| Stimulierung der Proteoglykan-Synthese in der Agarose-Zellkultur | |
|---|---|
| Verbindung nach Beispiel | Stimulationsfaktor der Proteoglykansynthese |
| 1 | 1,4 |
| 2 | 1,2 |
| 6 | 1,2 |
| 8 | 1,2 |
| 22 | 1,2 |
| 30 | 1,4 |
| 34 | 1,2 |
| 36 | 1,2 |
| 38 | 1,7 |
| 49 | 1,2 |
| 51 | 1,6 |
| 52 | 1,7 |
| 57 | 1,5 |
| 58 | 2,5 |
| 61 | 2,9 |
| 62 | 1,2 |
| 65 | 1,3 |
| 78 | 1,4 |
| 79 | 1,6 |
| Diacerein | 1,0 |

### 2. Wirksamkeit in der chondrozytären Chondrolyse, Prüfung in der Chondrozyten-Kultur

Zellen werden gewonnen und plattiert wie in Experiment 1. Medium: Dem kompletten Medium wie in Test 1 wird zusätzlich ab Behandlungsbeginn 5 u/ml humanes rec. Interleukin-1 alpha (IL-1, Sigma) zugesetzt, das wie die Testsubstanz bei jedem Mediumwechsel erneut zugefügt wird.

Versuchs-Durchführung: Die Behandlung erfolgt vom 5. bis 13. Tag der Primärkultur, am 12. Tag wird 20 µCi/ml Na₂³⁵SO₄ für 24 h dem Medium zugefügt. Die dissoziative Extraktion der Proteoglykane sowie Messung der Sulfatinkorporation wird wie in Test 1 durchgeführt.

Auswertung: IL-1 führt zu einer Synthesehemmung sowie zu einer Degradationssteigerung der Proteoglykane, was sich im geringeren Gehalt an markierten Matrixmolekülen in der Agarose-Schicht widerspiegelt. Der Stimulationsfaktor bezieht sich daher auf den Mittelwert der IL-1 behandelten Kontrolle.

Ergebnisse: Die Ergebnisse sind in Tabelle III aufgeführt.

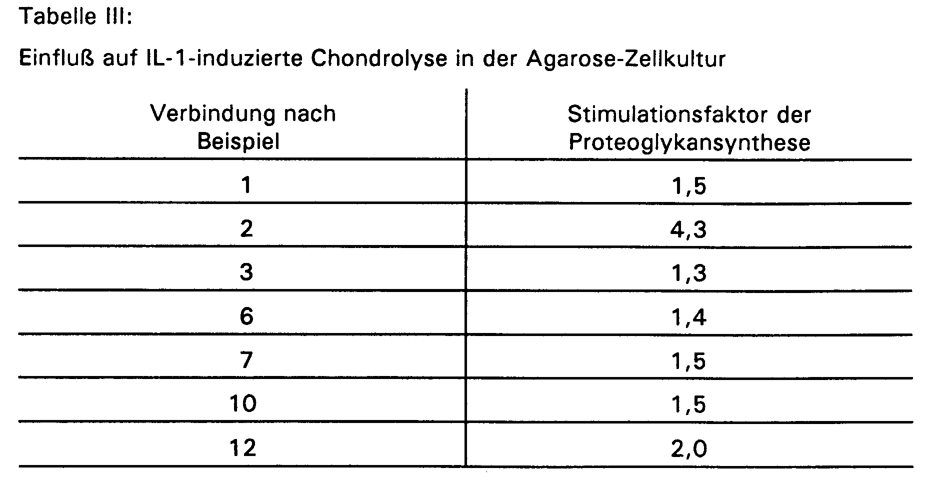

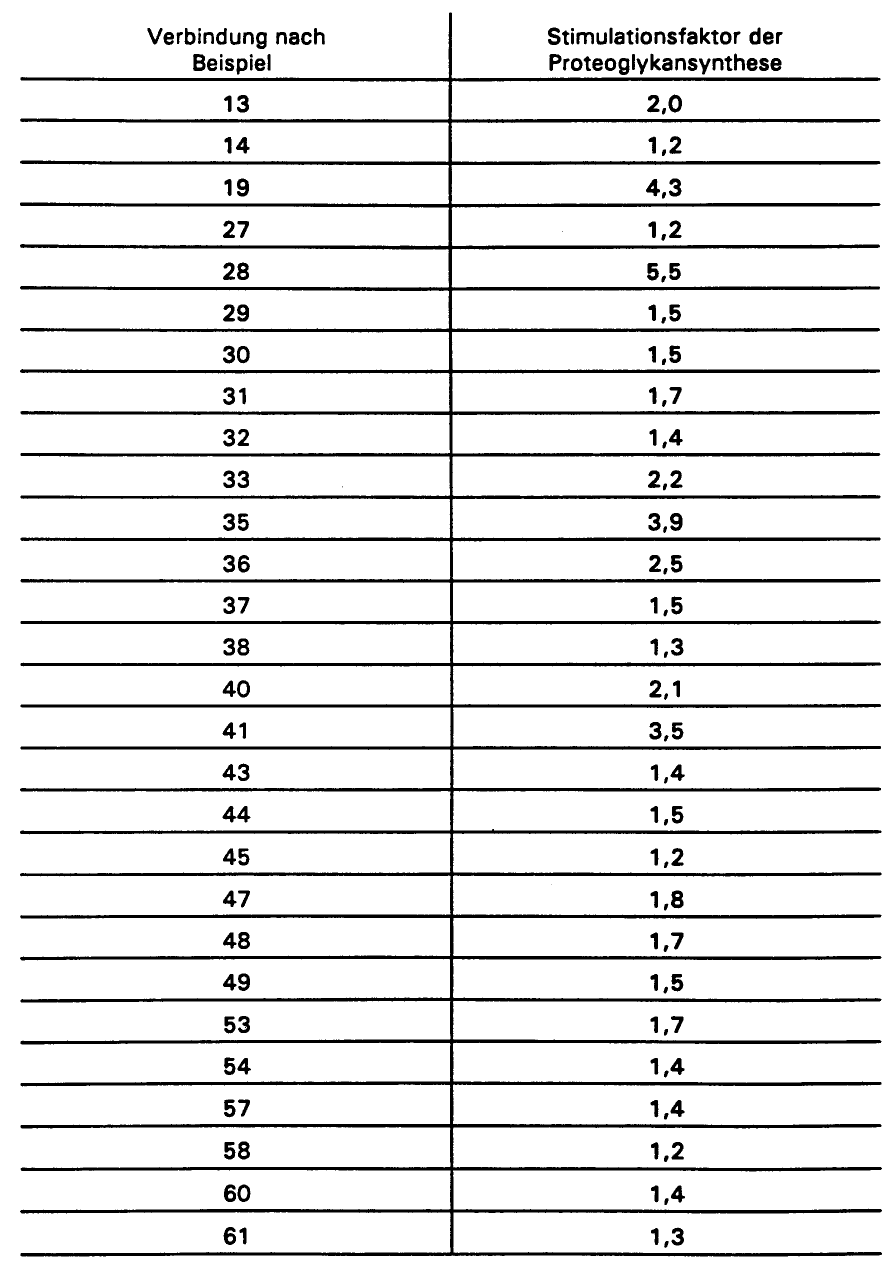

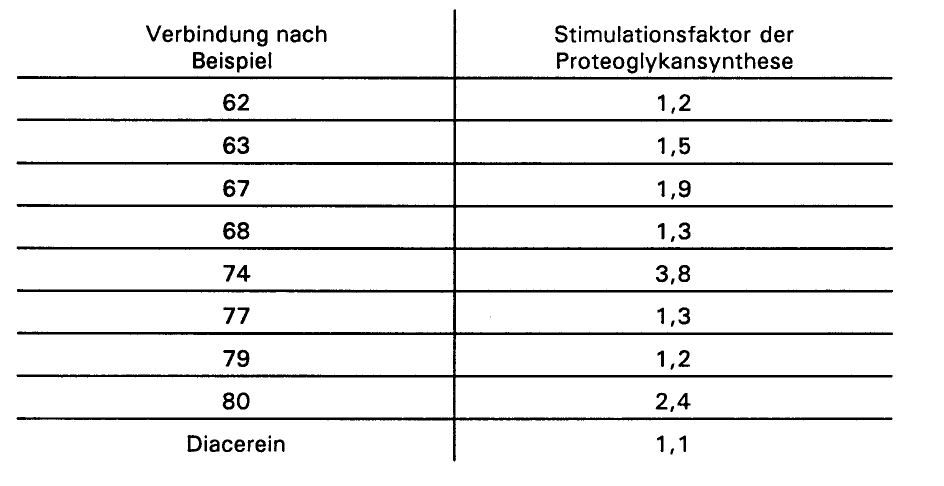

### 3. Inhibierung von Matrix Metalloproteasen (MMP)

Die erfindungsgemäßen Verbindungen zeigten deutliche inhibitorische Wirkungen auf proteolytische Enzyme, die sogenannten Matrix Metalloproteasen. Dies ist von großer Bedeutung, weil diese dem Fachmann an sich bekannten Enzyme am proteolytischen Abbau der intakten Knorpelmatrix entscheidend beteiligt sind.

### Zellkultur:

Kaninchen Synoviocyten (HIG-82; ATCC, Rockville, Maryland, USA) werden in Nährmedium HAM'S F12 (Sigma, Deisenhofen, Deutschland, Katalog Nr. N-6760) mit 10 % fötalem Rinder Serum (Sigma, Deisenhofen, Deutschland, Katalog Nr. F-2442), zusammen mit Penicillin 100 U/ml sowie Streptomycin 100 µg/ml, kultiviert. Nach konfluentem Zellwachstum wird die MMP Expression in serumfreiem HAM'S F12 durch Zugabe von 0,3 µmol/l Phorbol-12-Myristat-13-Acetat induziert. Nach 20 h Inkubationszeit bei 37°C wird der Überstand abgenommen.

### Aktivierung der MMP:

Der Überstand wird mit Trypsin (5 µg/ml) aktiviert. Nach 15 Minuten (min) bei 37°C wird die Aktivierung durch Zugabe von 1 mmol/l Phenylmethylsulfonylfluorid (PMSF) beendet und für weitere 10 min nachinkubiert. Das Gesamtvolumen des Ansatzes beträgt 210 µl.

### Messung der MMP Aktivität (Lit.: C.G. Knight et al.; FEBS Lett. 296, 263 (1992)):

20 µl des obengenannten Überstandes werden 1:10 verdünnt, und mit 240 µl Puffer (0,1 Tris/HCl pH 7,5; 0,1 M NaCl; 0,01 M CaCl₂; 0,05 % Brij) gemischt. Die Prüfsubstanz wird in der angegebenen Konzentration (s. Tabelle) zugegeben. Nach einer Inkubationszeit von 15 min wird die Reaktion durch Zugabe von 20 µmol/l Fluoreszenz-Substanz ((7-Methoxycoumarin-4-yl)acetyl-pro-Leu-Gly-Leu-[3-(2',4'-dinitrophenyl)-L-2,3-diaminopropionyl]-Ala-Arg-NH₂; Bachem, Heidelberg, Deutschland, Kat. Nr.: M-1895) gestartet. Die Reaktion wird nach 30 min durch Zugabe von 10 mmol/l EDTA beendet. Das Gesamtvolumen des Ansatzes beträgt 320 µl. Als Meßparameter dienen die Fluoreszenzintensitäten bei λₑₓ: 328 nm und λₑₓ: 393 nm. Um die mögliche Eigenfluoreszens der Prüfsubstanzen zu berücksichtigen, werden die Fluoreszenzintensitäten von Parallel-Messungen ohne Substrat von den Messwerten mit Substrat abgezogen. Alle Operationen erfolgen bei 20°C.
Im Kontrollexperiment ohne Hemmstoff wird die Fluoreszenz entsprechen 0 % Hemmung gesetzt, während völlige Auslöschung der Fluoreszenz 100 % Hemmung bedeutet.

| Hemmung der MMP | | | |
|---|---|---|---|
| Beispiel 3 | 83 % | Hemmung bei | 100 µM |
| | 20 % | | 30 µM |
| | 3 % | | 10 µM |
| Beispiel 22 | 34 % | | 100 µM |
| | 12 % | | 30 µM |
| Beispiel 53 | 25 % | | 100 µM |
| | 11 % | | 30 µM |
| Beispiel 59 | 43 % | | 30 µM |
| | 19 % | | 10 µM |
| Beispiel 62 | 83 % | | 100 µM |
| | 68 % | | 30 µM |
| | 23 % | | 10 µM |
| Beispiel 63 | 60 % | | 30 µM |
| | 26 % | | 10 µM |
| Beispiel 74 | 74 % | | 50 µM |
| | 47 % | | 30 µM |
| | 20 % | | 10 µM |

### 4. Hemmung der Mikrosomalen Lipidperoxidation

Beim unerwünschten Abbau der Knorpelmatrix sind in beträchtlichem Umfang auch oxidative Degradationsprozesse involviert. Die erfindungsgemäßen Verbindungen zeigten eine starke Hemmwirkung auf biologische Oxidationsprozesse, und sind deshalb zur Inhibierung des oxidativen Knorpelabbaus besonders geeignet.

### Gewinnung von Rattenlebermikrosomen:

Alle Schritte werden bei 0°C durchgeführt. Die Leber einer Ratte wird, um alles Hämoglobin zu entfernen, mit 0,9 % NaCl-Lösung gründlich gespült. Die grob zerkleinerte Leber wird dann in 10 mM Tris/HCl pH 7,4; 250 mM Saccharose (10 ml Puffer/g Leber) gepottert. Zunächst wird 5 Minuten bei 600xg zentrifugiert. Der Überstand wird dann 10 min bei 12000xg zentrifugiert und wird mit festem CaCl₂ (117,6 mg/100 ml) auf eine Konzentration von 8 mM eingestellt. Durch Zentrifugieren bei 25000 xg (15 min) wird ein Mikrosomenniederschlag gewonnen. Dieser Niederschlag wird im gleichen Volumen Puffer (10 mM Tris/HCl pH 7,4; 150 mM KCl) rehomogenisiert und erneut 15 min bei 25000 xg zentrifugiert.

### Peroxidation von Rattenlebermikrosomen:

Ein Testansatz besteht aus 20 µl Mikrosomen (100 mg/ml), vgl. oben, gelöst in Puffer (250 mM Tris/HCl pH 6,6, 750 mM KCl), 10 µl 50 mM MgCl₂, 10 µl 200 mM Isocitronensäure, 10 µl 4mM NADP (3,028 mg/ml Wasser), 10 µl 25 mM Niacinamid, 10 µl Isocitratdehydrogenase (1:100 verdünnt) und 20 µl Wasser oder Testsubstanz. Gestartet wird die Reaktion mit 10 µl 0,25 mM FeSO₄. Die Inkubation dauert 10 Minuten und erfolgt bei 37°C. Gestoppt wird mit 500 µl eiskalter 20 %iger Trichloressigsäure. Entstandenes Malondialdehyd wird durch Zugabe von 500 µl 0,67 %iger Thiobarbitursäure und 30 Minuten Inkubation bei 90°C in eine pinkfarbene Verbindung überführt, die bei 532 nm gemessen wird. Die Extinktion im Kontrollansatz ohne Prüfpräparat wird auf 0 % Hemmung gesetzt, während das völlige Verschwinden des Signals 100 % Hemmung bedeutet.

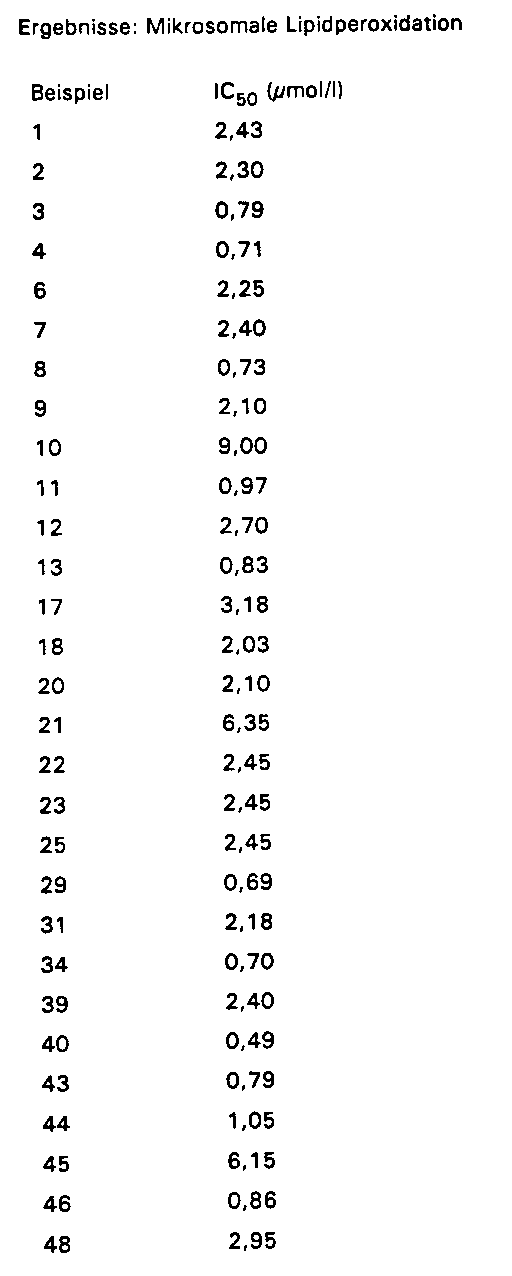

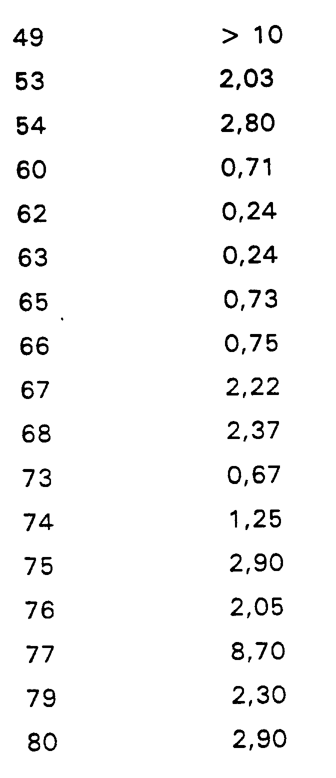

### 5. Freisetzung von Interleukinen (insbesondere IL-1β) aus humanen mononuklearen Zellen

Die erfindungsgemäßen Verbindungen haben eine starke Hemmwirkung auf die Freisetzung von Interleukinen aus Humanzellen. Dies ist von großer pharmakologischer Bedeutung weil Interleukine die unerwünschte Degradation der Knorpelmatrix auslösen können.

### Gewinnung mononuklearer Zellen aus Humanblut

10 ml Humanblut, stabilisiert mit 1 ml 3,8 %iger Natriumcitrat-Lösung, werden mit 10 ml PM16 (Serva, Heidelberg) verdünnt und mit 15 ml Lymphoprep (Dr. Molter GmbH, Heidelberg) unterschichtet. Die Proben werden 40 Minuten bei 400 xg (1600 rpm Minifuge 2, Heraeus, Osterode) zentrifugiert (Raumtemperatur). Die mononuklearen Zellen sind als weißer Ring an der Grenze Lymphoprep/Plasma sichtbar. Dieser Ring wird mit einer Spritze vorsichtig entnommen, mit dem gleichen Volumen PM16 verdünnt und 10 Minuten bei 400 xg zentrifugiert. Der Niederschlag wird mit ∼ 10 ml RPMI 1640 (+ 300 mg/l L-Glutamin, Gibco, Eggenstein) gewaschen. Nach Resuspendieren der Zellen in ∼ 1 ml RPMI 1640 (+ 300 mg/l L-Glutamin, + 25 mM HEPES, + 100 µg/ml Streptomycin, + 100 µg/ml Penicillin) wird die Zelldichte mit einem Coulter Counter JT (Coulter Diagnostics) bestimmt und auf 5 * 10⁶/ml eingestellt. Die Zellen bestehen typischerweise aus 90 % Lymphozyten und 10 % Monozyten.

### Stimulierung der Freisetzung von Zytokinen, insbesondere Interleukin 1β

230 µl mononukleare Zellen werden mit 10 µl Testsubstanz (10 µM in DMSO/Wasser 1/10) und 10 µl Lipopolysaccharide (500 µg gelöst in 1 ml Dimethylsulfoxid (DMSO) und vor Testbeginn 1/10 mit Wasser verdünnt, von Salmonella abortus equi, Sigma, Deisenhofen) 20-22 Stunden bei 37°C; 5 % CO₂ inkubiert. Die Proben werden in einem Eisbad auf 0°C abgekühlt und in einer Sigma-Zentrifuge zentrifugiert (2 Minuten; 2000 rpm). Aliquote des Überstandes werden mit einem kommerziell erhältlichen ELISA (Biermann, Bad Nauheim) bestimmt.

### Ergebnisse:

| Hemmung der Interleukin-Freisetzung (alle Beispielsubstanzen wurden bei 10 µmol/l geprüft) | | |
|---|---|---|
| Beispiel | % Hemmung | Interleukin |
| 7 | 21 | IL-1alpha |
| 22 | 80 | I-1alpha |
| 22 | 86 | TNF-alpha |
| 23 | 75 | IL-1alpha |
| 23 | 70 | TNF-alpha |
| 25 | 60 | IL-1alpha |
| 25 | 36 | TNF-alpha |
| 31 | 32 | IL-1beta |
| 45 | 22 | IL-1alpha |
| 53 | 53 | IL-1beta |
| 60 | 24 | TNF-alpha |
| 61 | 24 | IL-1beta |
| 62 | 46 | IL-1beta |
| 63 | 31 | IL-1beta |
| 67 | 26 | IL-1beta |
| 80 | 21 | TNF-alpha |

## Patentansprüche

1. Arzneimittel, enthaltend eine Verbindung der Formel I 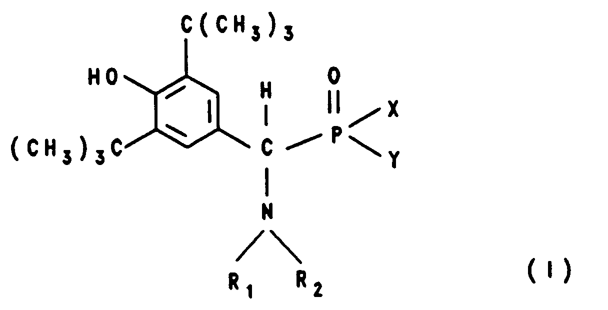 und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₁₂)-Alkyl,
c) (C₁-C₁₂)-Alkyl, ein- bis mehrfach unabhängig voneinander substituiert durch
1) Phenyl,
2) Phenyl, ein- bis dreifach unabhängig voneinander substituiert durch
2.1. Fluor-, Chlor-, Brom- oder Jodatom,
2.2. (C₁-C₃)-Alkyl,
2.3. (C₁-C₃)-Alkoxy,
2.4. Methylendioxy,
2.5. Nitro,
2.6. Amino,
2.7. Amidino,
2.8. Guanidino,
2.9. Hydroxy,
2.10. (C₁-C₆)-Alkoxycarbonyl oder
2.11. Cyano,
3) einen monocyclischen 5- oder 6-gliedrigen gesättigten heterocyclischen Ring,
4) Amino, unsubstituiert oder ein- bis zweimal substituiert durch (C₁-C₃)-Alkyl, oder
5) -C(O)-O-(C₁-C₃)-Alkyl,
d) Tetralin,
e) Phenyl,
f) Phenyl, ein- bis dreifach unabhängig voneinander substituiert durch
1) (C₁-C₄)-Alkyl,
2) (C₁-C₄)-Alkyl, ein- oder mehrfach substituiert durch Fluor-, Chlor-, Brom- oder Jodatom,
3) Fluor-, Chlor-, Brom- oder Jodatom,
4) Nitro,
5) Aminosulfonyl,
6) Amidino,
7) Guanidino,
8) (C₁-C₃)-Alkyoxy,
9) Methylendioxy,
10) Hydroxy,
11) Carboxy,
12) Cyano,
13) (C₁-C₃)-Alkyoxycarbonyl,
14) - CH = CH - COOH,
15) 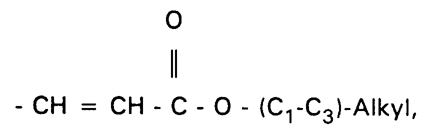
16) Amino oder
17) Amino, substituiert durch den Rest der Formel II, 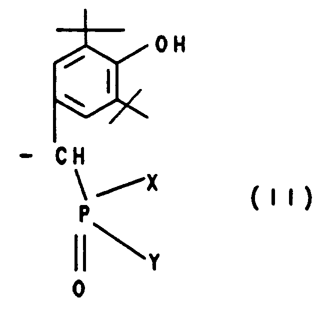 worin X und Y unabhängig voneinander
17.1. Wasserstoffatom,
17.2. (C₁-C₆)-Alkyl,
17.3. (C₁-C₆)-Alkoxy,
17.4. (C₃-C₆)-Alkenyloxy,
17.5. Phenoxy oder
17.6. Hydroxy bedeutet,
g) Naphthyl,
h) Naphthyl, ein- bis mehrfach substituiert wie unter f)1) bis f)17) definiert,
i) Heteroaryl,
k) Heteroaryl, ein- bis mehrfach substituiert durch Phenyl, Benzyl oder wie unter f)1) bis f)17) definiert, steht, oder
l) R¹ und R² bilden zusammen mit dem Stickstoffatom, an das sie gebunden sind, einen 4- bis 7-gliedrigen Ring,
m) R¹ und R² bilden zusammen mit dem Stickstoffatom, an das sie gebunden sind, einen 5- bis 7-gliedrigen Ring, worin ein ringständiges C-Atom durch ein Sauerstoffatom oder ein Stickstoffatom ersetzt ist, oder
n) R¹ und R² bilden zusammen mit dem Stickstoffatom, an das sie gebunden sind, einen 5- bis 7-gliedrigen Ring, worin ein ringständiges C-Atom durch ein Stickstoffatom ersetzt ist und dieses Stickstoffatom ist substituiert durch
1) Phenyl oder
2) den Rest der Formel II, worin X und Y unabhängig voneinander die unter f)17.1. bis f)17.6. genannte Bedeutung haben, und
X und Y gleich oder verschieden sind und unabhängig voneinander die unter f)17.1. bis f)17.6. genannte Bedeutung haben;
und einen physiologisch verträglichen Träger.

2. Arzneimittel nach Anspruch 1, dadurch gekennzeichnet, daß man mindestens eine Verbindung der Formel I und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₁₂)-Alkyl,
c) (C₁-C₁₂)-Alkyl, ein- bis mehrfach unabhängig voneinander substituiert durch
1) Phenyl,
2) Phenyl, ein- bis dreifach unabhängig voneinander substituiert durch
2.1. Fluor-, Chlor-, Brom- oder Jodatom,
2.2. (C₁-C₃)-Alkyl,
2.3. (C₁-C₃)-Alkoxy,
2.4. Methylendioxy,
2.5. Nitro,
2.6. Amino,
2.7. Amidino,
2.8. Guanidino,
2.9. Hydroxy,
2.10. (C₁-C₆)-Alkoxycarbonyl oder
2.11. Cyano,
3) Piperazinyl oder
4) Morpholinyl,
d) Phenyl,
e) Phenyl, ein- bis dreifach unabhängig voneinander substituiert durch
1) (C₁-C₄)-Alkyl,
2) (C₁-C₄)-Alkyl, ein- bis mehrfach substituiert durch Fluor-, Chlor-, Brom- oder Jodatom,
3) Fluor-, Chlor-, Brom- oder Jodatom,
4) Nitro,
5) Aminosulfonyl,
6) Amidino,
7) Guanidino,
8) (C₁-C₃)-Alkyloxy,
9) Methylendioxy,
10) Hydroxy,
11) Carboxy,
12) Cyano,
13) (C₁-C₃)-Alkyoxycarbonyl,
14) - CH = CH - COOH,
15) 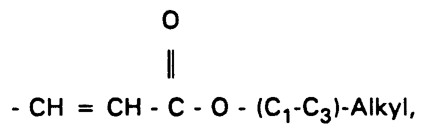
16) Amino oder
17) Amino, substituiert durch den Rest der Formel II, worin X und Y unabhängig voneinander
17.1. Wasserstoffatom,
17.2. (C₁-C₆)-Alkyl,
17.3. (C₁-C₆)-Alkoxy,
17.4. (C₃-C₆)-Alkenyloxy,
17.5. Phenoxy oder
17.6. Hydroxy bedeuten,
f) (C₅-C₇)-Cycloalkyl,
g) (C₅-C₇)-Cycloalkyl, ein- bis mehrfach unabhängig voneinander substituiert durch die unter c)1) bis c)4) genannten Reste,
h) Adamantyl,
i) (C₁-C₁₂)-Alkyl, substituiert durch Amino, unsubstituiert oder ein- bis zweimal substituiert durch (C₁-C₃)-Alkyl,
j) (C₁-C₁₂)-Alkyl substituiert durch -C(O)-O-(C₁-C₃)-Alkyl,
k) Naphthyl,
l) Tetralin,
m) Pyridyl,
n) Pyridyl, ein- bis mehrfach unabhängig voneinander substiuiert wie unter e)1) bis e)17) definiert,
o) Pyrimidyl,
p) Pyrimidyl, ein- bis mehrfach unabhängig voneinander substituiert wie unter e)1) bis e)17) definiert, steht,
q) Piperidylrest, unsubstituiert oder ein- bis vierfach substituiert durch
1) (C₁-C₄)-Alkyl,
2) Phenyl oder
3) Benzyl, steht, oder
r) R¹ und R² bilden zusammen mit dem Stickstoffatom, an das sie gebunden sind, einen Piperidinorest,
s) R¹ und R² bilden zusammen mit dem Stickstoffatom, an das sie gebunden sind, einen Piperazino- oder Morpholinorest, oder
t) R¹ und R² bilden zusammen mit dem Stickstoffatom, an das sie gebunden sind, einen Piperazinorest, der am Stickstoffatom substituiert ist durch
1) Phenyl oder
2) den Rest der Formel II, worin X und Y unabhängig voneinander die unter e)17.1. bis e)17.6. genannte Bedeutung haben, und
X und Y gleich oder verschieden sind und unabhängig voneinander die unter e)17.1. bis e)17.6. genannte Bedeutung haben;
und einen physiologisch verträglichen Träger einsetzt.

3. Arzneimittel nach den Ansprüchen 1 oder 2, dadurch gekennzeichnet, daß man mindestens eine Verbindung der Formel I und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₅)-Alkyl,
c) (C₁-C₅)-Alkyl, ein- bis mehrfach unabhängig voneinander substituiert durch
1) Phenyl,
2) Phenyl, ein- bis dreifach unabhängig voneinander substituiert durch
2.1. Fluor oder Chloratom,
2.2. Methyl, Ethyl oder Propyl,
2.3. Methoxy,
2.4. Methylendioxy,
2.5. Nitro,
2.6. Amino,
2.7. Amidino,
2.8. Guanidino,
2.9. Hydroxy,
2.10. Methoxycarbonyl oder
2.11. Cyano, oder
3) Morpholinyl,
d) (C₅-C₇)-Cycloalkyl,
e) (C₅-C₇)-Cycloalkyl, ein- bis dreifach unabhängig voneinander substituiert wie unter c)1) bis c)3) definiert,
f) Adamantyl,
g) Naphthyl,
h) Phenyl,
i) Phenyl, ein- bis dreifach unabhängig voneinander substituiert durch
1) (C₁-C₂)-Alkyl,
2) Trifluormethyl,
3) Fluor- oder Chloratom,
4) Nitro,
5) Aminosulfonyl,
6) Amidino,
7) Guanidino,
8) Methoxy,
9) Methylendioxy,
10) Hydroxy,
11) Carboxy,
12) Cyano,
13) Methoxycarbonyl,
14) - CH = CH - COOH,
15) 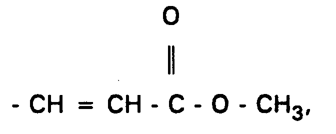
16) Amino oder
17) Amino, substituiert durch den Rest der Formel II worin X und Y unabhängig voneinander
17.1. Wasserstoffatom,
17.2. Methoxy, Ethoxy, Propoxy oder Butyloxy
17.3. Allyl,
17.4. Phenoxy oder
17.5. Hydroxy bedeuten,
k) Pyridyl,
l) Pyrimidyl steht, oder
m) R¹ und R² bilden zusammen mit dem Stickstoffatom, an das sie gebunden sind, einen Piperidinorest,
n) R¹ und R² bilden zusammen mit dem Stickstoffatom, an das sie gebunden sind, einen Piperazino- oder Morpholinorest, oder
o) R¹ und R² bilden zusammen mit dem Stickstoffatom, an das sie gebunden sind, einen Piperazinorest, der am Stickstoffatom substituiert ist durch
1) Phenyl oder
2) den Rest der Formel II, worin X und Y unabhängig voneinander die unter i)17.1. bis i)17.5. genannte Bedeutung haben, und
X und Y gleich oder verschieden sind und unabhängig voneinander die unter i)17.1. bis i)17.6. genannte Bedeutung haben;
und einen physiologisch verträglichen Träger einsetzt.

4. Arzneimittel nach einem oder mehreren der Ansprüche 1 bis 3, dadurch gekennzeichnet, daß man mindestens eine Verbindung der Formel I und/oder ein physiologisch verträgliches Salz der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) Phenyl,
c) Phenyl, ein- bis dreifach unabhängig voneinander substituiert durch
1) Fluor- oder Chloratom,
2) Methylendioxy oder
3) (C₁-C₃)-Alkoxy, steht und
X und Y unabhängig voneinander
1) Wasserstoffatom,
2) (C₁-C₃)-Alkoxy oder
3) Hydroxy, bedeutet;
und ein physiologisch verträglicher Träger einsetzt.

5. Verbindung der Formel I gemäß Anspruch 1 und/oder physiologisch verträgliche Salze der Verbindung der Formel I und/oder eine gegebenenfalls stereoisomere Form der Verbindung der Formel I, wobei
R¹ und R² gleich oder verschieden sind und unabhängig voneinander für
a) Wasserstoffatom,
b) (C₁-C₁₂)-Alkyl, ein- bis mehrfach unabhängig voneinander substituiert durch
1) Phenyl, ein- bis dreifach unabhängig voneinander substituiert durch
1.1. Fluor-, Chlor-, Brom- oder Jodatom,
1.2. (C₁-C₃)-Alkyl,
1.3. (C₁-C₃)-Alkoxy,
1.4. Methylendioxy,
1.5. Nitro,
1.6. Amino,
1.7. Amidino,
1.8. Guanidino,
1.9. Hydroxy,
1.10. (C₁-C₆)-Alkoxycarbonyl oder
1.11. Cyano,
2) einen monocyclischen 5- oder 6-gliedrigen gesättigten heterocyclischen Ring,
3) Amino, unsubstituiert oder ein- bis zweimal substituiert durch (C₁-C₃)-Alkyl, oder
4) -C(O)-O-(C₁-C₃)-Alkyl,
c) Tetralin,
d) Phenyl, ein- bis dreifach unabhängig voneinander substituiert durch
1) (C₁-C₄)-Alkyl,
2) (C₁-C₄)-Alkyl, ein- bis mehrfach substituiert durch Fluor-, Chlor-, Brom- oder Jodatom,
3) Nitro,
4) Aminosulfonyl,
5) Amidino,
6) Guanidino,
7) (C₁-C₃)-Alkyloxy,
8) Methylendioxy,
9) Hydroxy,
10) Carboxy,
11) Cyano,
12) (C₁-C₃)-Alkyoxycarbonyl,
13) - CH = CH - COOH,
14) 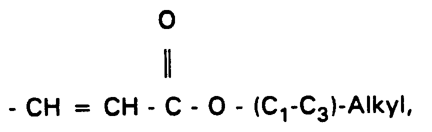
15) Amino oder
16) Amino, substituiert durch den Rest der Formel II gemäß Anspruch 1, worin X und Y unabhängig voneinander
16.1. Wasserstoffatom,
16.2. (C₁-C₆)-Alkyl,
16.3. (C₁-C₆)-Alkoxy,
16.4. (C₃-C₆)-Alkenyloxy,
16.5. Phenoxy oder
16.6. Hydroxy bedeuten,
e) Naphthyl, ein- bis mehrfach substituiert wie unter d)1) bis d)16) definiert,
f) Heteroaryl,
g) Heteroaryl, ein- bis mehrfach substituiert durch Phenyl, Benzyl oder wie unter d)1) bis d)16) definiert, steht, oder
h) R¹ und R² bilden zusammen mit dem Stickstoffatom, an das sie gebunden sind, einen 5- bis 7-gliedrigen Ring, worin ein ringständiges C-Atom durch ein Sauerstoffatom oder ein Stickstoffatom ersetzt ist, oder
i) R¹ und R² bilden zusammen mit dem Stickstoffatom, an das sie gebunden sind, einen 5- bis 7-gliedrigen Ring, worin ein ringständiges C-Atom durch ein Stickstoffatom ersetzt ist und dieses Stickstoffatom ist substituiert durch
1) Phenyl oder
2) den Rest der Formel II, worin X und Y unabhängig voneinander die unter d)16.1. bis d)16.6. genannte Bedeutung haben, und
X und Y gleich oder verschieden sind und unabhängig voneinander die unter d)16.1. bis d)16.6. genannte Bedeutung haben; oder wobei
R¹ für Phenyl unsubstituiert oder ein- bis dreifach substituiert durch Fluor, Chlor-, Brom- oder Jodatom, steht
R2 für Wasserstoffatom steht und
X und Y unabhängig voneinander für
1) Wasserstoffatom,
2) (C₁-C₆)-Alkyl,
3) (C₃-C₆)-Alkenyloxy,
4) Phenoxy oder
5) Hydroxy stehen.

6. Verbindung der Formel I gemäß Anspruch 5, dadurch gekennzeichnet, daß
R¹ und R² gleich oder verschieden sind und unabhängig voneinander für
a) Wasserstoffatom,
b) (C₁-C₁₂)-Alkyl, ein- bis mehrfach unabhängig voneinander substituiert durch
1) Phenyl, ein- bis dreifach unabhängig voneinander substituiert durch
1.1. Fluor-, Chlor-, Brom- oder Jodatom
1.2. Methylendioxy oder
1.3. Cyano,
2) Amino, unsubstituiert oder ein- bis zweimal substituiert durch (C₁-C₃)-Alkyl, oder
3) -C(O)-O-(C₁-C₃)-Alkyl,
c) Phenyl, ein- bis dreifach unabhängig voneinander substituiert durch
1) (C₁-C₄)-Alkyl, ein- bis mehrfach substituiert durch Fluor-, Chlor-, Brom- oder Jodatom,
2) (C₁-C₃)-Alkyloxy,
3) Methylendioxy,
4) Cyano oder
5) Amino, substituiert durch den Rest der Formel II, worin X und Y unabhängig voneinander
5.1. Wasserstoffatom,
5.2. (C₁-C₆)-Alkyl,
5.3. (C₁-C₆)-Alkoxy,
5.4. (C₃-C₆)-Alkenyloxy,
5.5. Phenoxy oder
5.6. Hydroxy bedeuten,
d) Pyridyl oder
e) Pyrimidyl steht, und worin
X und Y unabhängig voneinander die unter c)5.1 bis c)5.6 genannte Bedeutung haben; oder wobei
R¹ für Phenyl oder Phenyl, ein- bis dreifach substituiert durch Fluor- oder Chloratom, steht,
R2 für Wasserstoffatom steht und
X und Y unabhängig voneinander für
1) Wasserstoffatom,
2) (C₁-C₂)-Alkyl oder
3) Hydroxy stehen.

7. Verfahren zur Herstellung der Verbindung der Formel I, dadurch gekennzeichnet, daß man
A) einen Phosphorigsäurediester oder Phosphorigsäuremonoester der Formel III, 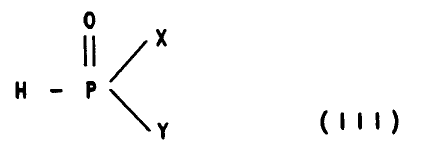 wobei X und Y unabhängig voneinander für
a) Wasserstoffatom,
b) (C₁-C₆)-Alkyl,
c) (C₁-C₆)-Alkoxy,
d) (C₃-C₆)-Alkenyloxy oder
e) Phenoxy stehen,
mit einem Azomethin der Formel IV, 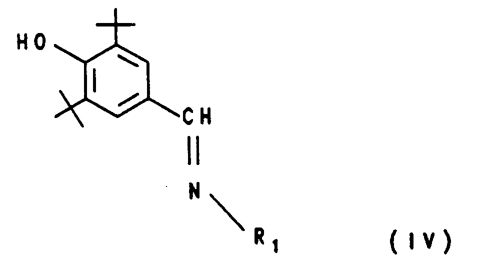 wobei R¹ die unter der Formel I gemäß Anspruch 1 für b) bis k genannte Bedeutung hat,
zu einer Verbindung der Formel Ia umsetzt, 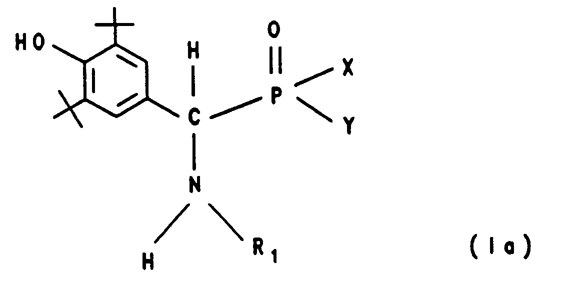 wobei R¹ die in Formel IV und X und Y die in Formel III angegebene Bedeutung haben, oder
B) eine Verbindung der Formel NHR¹R²,
wobei R¹ und R² unabhängig voneinander die in Formel I angegebene Bedeutung haben,
in Gegenwart von 4-Hydroxy-3,5-di-tert.butylbenzaldehyd und einer Verbindung der Formel III zu einer Verbindung der Formel I umsetzt, oder
C) eine Additionsverbindung der Formel V 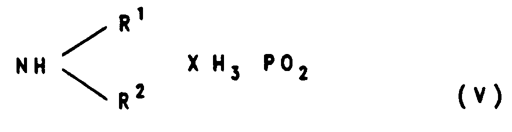 wobei R¹ und R² unabhängig voneinander die in Formel I angegebene Bedeutung haben,
in Gegenwart von 4-Hydroxy-3,5-di-tert.butylbenzaldehyd zu einer Verbindung der Formel Ib 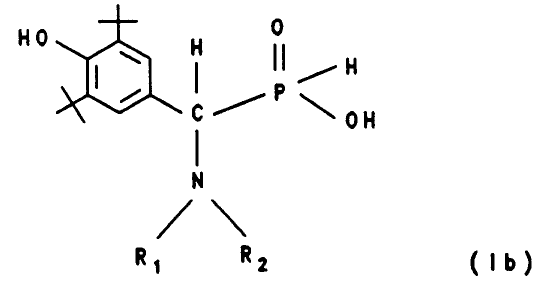 umsetzt, oder
D) eine Verbindung der Formel I, wobei X und Y unabhängig voneinander für
a) (C₁-C₆)-Alkyl,
b) (C₁-C₆)-Alkoxy,
c) (C₃-C₆)-Alkenyloxy oder
d) Phenoxy, stehen
zu einer Verbindung der Formel I, wobei X und Y unabhängig voneinander für Hydroxy stehen, hydrolysiert, oder
E) eine nach Verfahren A) - D) hergestellte Verbindung der Formel I, die aufgrund ihrer chemischen Struktur in enantiomenren Formen auftritt, durch Salzbildung mit enantiomerenreinen Säuren oder Basen, Chromatographie an chiralen Stationärphasen oder Derivatisierung mittels chiraler enantiomerenreiner Verbindungen wie Aminosäuren, Trennung der somit erhaltenen Diastereomeren, und Abspaltung der chiralen Hilfsgruppe in die reinen Enantiomeren auftrennt, oder
F) die nach Verfahren A) - E) hergestellte Verbindung der Formel I entweder in freier Form isoliert oder im Falle des Vorliegens von sauren oder basischen Gruppen gegebenenfalls in physiologisch verträgliche kristalline Salze umwandelt.

8. Verwendung von mindestens einer Verbindung der Formel I gemäß einem oder mehreren der Ansprüche 1 bis 4, zur Herstellung von Arzneimitteln zur Prophylaxe und Therapie von degenerativen Gelenkserkrankungen.

9. Verwendung nach Anspruch 8, für die Behandlung von Arthrose, Erkrankungen des rheumatischen Formenkreises mit Knorpelabbau, chronischer Polyarthritis, Knorpelschwund nach Gelenktrauma oder Knorpelschwund nach längerer Stillegung von Gelenken.

10. Verfahren zur Herstellung eines Arzneimittels gemäß der Ansprüche 1 bis 4, dadurch gekennzeichnet, daß man mindestens eine Verbindung der Formel I und/oder mindestens ein physiologisch verträgliches Salz der Verbindung der Formel I und/oder eine gegebenenfalls stereoisomere Form der Verbindung der Formel I und/oder eine nach dem Verfahren nach Anspruch 7 erhaltene Verbindung mit physiologisch annehmbaren Hilfs- und Trägerstoffen und gegebenenfalls weiteren Zusatzstoffen und/oder anderen Wirkstoffen in eine geeignete Darreichungsform bringt.
